# EUROPEAN PATENT APPLICATION

(11) **EP 4 509 525 A1**
(43) Date of publication of application: **19.02.2025**
(21) Application number: 23788660.1
(22) Date of filing: 14.04.2023
(51) Int. Cl.: C07K 14/74, C07K 16/00, C12N 15/62, A61K 38/00, A61K 39/00

(54) **FUSION PROTEIN PLATFORM WITH IMPROVED HALF-LIFE**

(30) Priority: 15.04.2022 KR 20220047003
(71) Applicant: Imbiologics Corp., Suwon-si Gyeonggi-do 16229 (KR)
(72) Inventor: HA, Gyongsik, Seoul 07287 (KR); LEE, Chungmin, Seongnam-si, Gyeonggi-do 13629 (KR); LEE, Hong Jai, Ansan-si, Gyeonggi-do 15231 (KR); KANG, Sungmuk, Seoul 07279 (KR); YI, Jiye, Suwon-si, Gyeonggi-do 16509 (KR); CHO, Yonghyun, Yongin-si, Gyeonggi-do 17103 (KR)
(74) Representative: Rückerl, Florian
(86) International application number: PCT/KR2023/005133
(87) International publication number: WO 2023/200311

(57) **Abstract**

The present invention relates to a fusion protein platform with improved half-life and relates to a fusion protein platform using an IgG Fc region, IgM region, J chain and/or linker. In addition, it relates to a fusion protein produced through the platform, and specifically relates to a fusion protein comprising an IgG Fc region, an IgM region, a J chain, a linker and/or a biologically active molecule.

According to the fusion protein platform of the present invention, it is possible to select various biologically active molecules depending on the purpose and use various types of biologically active molecules in combination. Additionally, the biologically active molecules comprised in the platform can have increased avidity. Therefore, the fusion protein prepared through the platform has an excellent target recognition and control effect, and there is less risk of unpredictable side effects due to non-specific binding. In addition, the half-life in the blood is increased, and thus the treatment effect sought to be achieved through the fusion protein can be maximized.

## Description

### [Technical Field]

The present invention relates to a fusion protein platform with improved half-life and relates to a fusion protein platform using an IgG Fc region, IgM region, J chain and/or linker. In addition, it relates to a fusion protein produced through the platform, and specifically relates to a fusion protein comprising an IgG Fc region, an IgM region, a J chain, a linker and/or a biologically active molecule.

### [Background Art]

Various types of biologically active molecules are used for the purpose of treating diseases. The activity of biologically active molecules is directly related to the therapeutic effect, and thus various approaches have been taken to increase their activity.

Biological activity is mainly achieved through the process of transmitting signals by binding active molecules, including ligands, to receptors present in living organisms. One way to enhance the therapeutic effects of a biologically active molecule is to increase the binding affinity or avidity of the active molecule with its target receptors, allowing the activation of receptors with lower concentrations of the active molecule within the organism.

Therefore, in order to achieve an increase in the binding avidity of active molecules, various methods have been developed, such as fusing the fragment crystallizable region (Fc region) of immunoglobulin G (IgG), which is one of the isotypes of immunoglobulins, to proteins, or utilizing biotin-streptavidin binding. However, the effect of increasing the binding avidity of the dimer Fc fusion protein is not significant. In the case of biotin-streptavidin-based materials, the multimer manufacturing process is complex and there are limitations in securing a quality that can be used as a medicine.

Accordingly, there is still a demand for the development of technology to increase the activity of biologically active molecules, or for protein drugs with improved therapeutic effects by enhancing the activity of biologically active molecules.

### [Technical Problem]

The inventors have conducted research to increase the half-life of multimeric fusion proteins in blood and minimize side effects and developed a platform capable of (1) imparting FcRn binding ability to a fusion protein comprising IgM Fc, and (2) minimizing the binding affinity of the fusion protein to an IgM-specific receptor. As a result, we have completed the present invention by experimentally proving that the fusion protein produced through the platform increases the avidity of biological molecules, maximizes the effect of increasing target binding ability and blood half-life, and minimizes the possibility of side effects due to non-specific binding.

### [Technical Solution]

Each description and embodiment of the present invention also applies to other descriptions and embodiments, and the scope of the present invention is not limited by the specific description set forth below.

In addition, terms that are not specifically defined in this specification have meanings commonly used in the technical field to which the present invention pertains. Additionally, unless specifically defined in context, singular includes plural and plural includes singular.

One aspect of the present invention provides a fusion protein, represented by the following structural formula:

[(X)ₐ-(Y)_{b}-(IgG Fc region)_{c}-(IgM Fc region)]ₘ-(Z)ₙ

wherein
X is a biologically active molecule,
Y is a linker,
IgG Fc region is a monomer,
IgM Fc region is a monomer,
Z is a J chain,
a is 1 or 2, b is 1 or 2, c is 0 or 1, m is 5 or 6, and n is 0 or 1.

In one embodiment according to the present invention, a is 2, b is 2, c is 1, m is 5, and n is 1. For example, when a is 2, b is 2, c is 1, m is 5, and n is 1, the fusion protein may be interchangeably expressed as [(X)₂-(Y)₂-(IgG Fc region)₁-(IgM Fc region)]₅-(Z)₁ or [(X)₂-(Y)₂-(IgG Fc region)-(IgM Fc region)]s-(Z).

In another embodiment according to the present invention, a is 2, b is 2, c is 0, m is 5, and n is 1. For example, when a is 2, b is 2, c is 0, m is 5, and n is 1, the fusion protein may be interchangeably expressed as [(X)₂-(Y)₂-(IgG Fc region)₀-(IgM Fc region)]₅-(Z)₁ or [(X)₂-(Y)₂-(IgM Fc region)]₅-(Z).

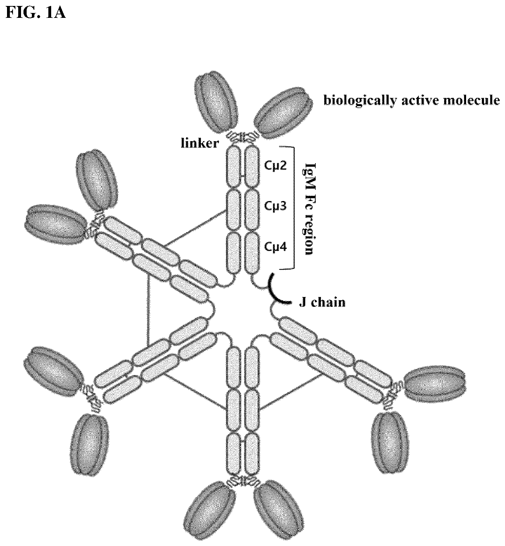

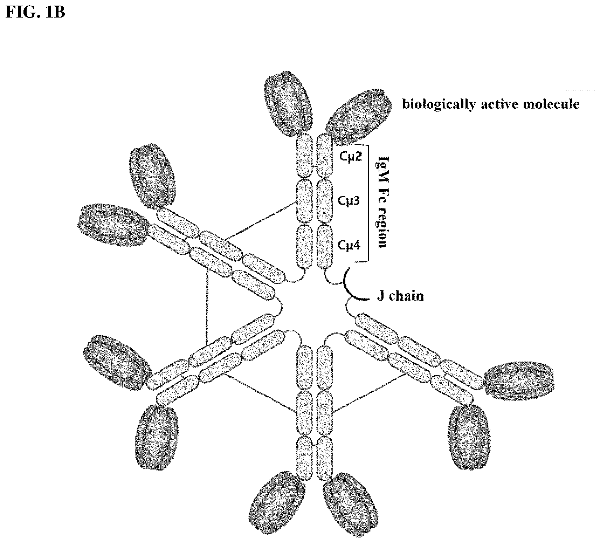

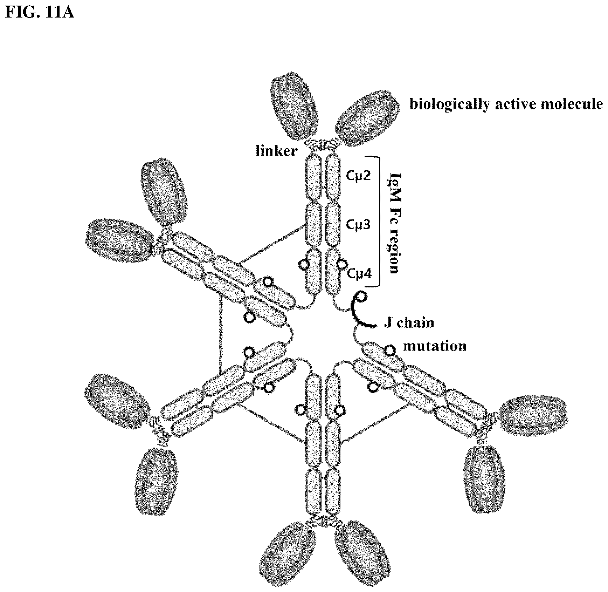

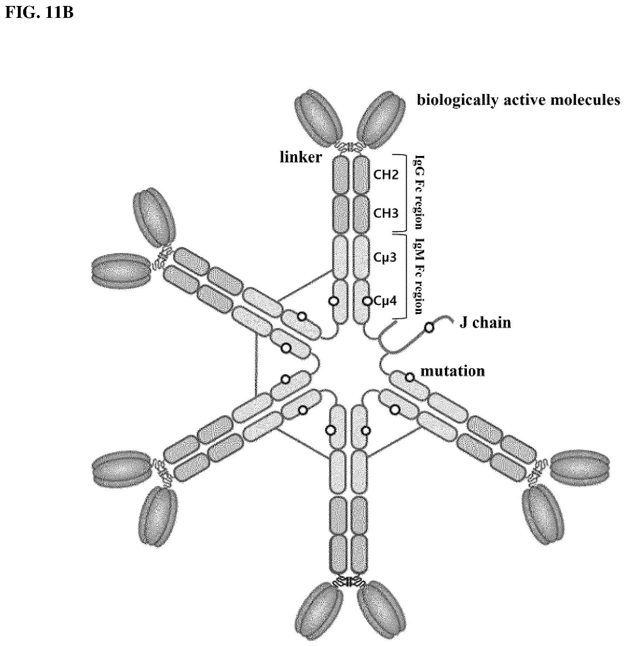

In addition, when m is 5, and n is 1, the fusion protein may be in a form in which the J chain is bonded to the first IgM Fc region monomer and the fifth IgM Fc region monomer by a disulfide bond, and representative structures thereof are shown in FIGs. 1A, 1B, 11A and 11B.

The fusion protein provided in the present invention has 10 to 12 binding domains through the IgM Fc regions to maximize the avidity of a biologically active molecule and solves the steric hindrance problem of the existing IgM antibody through the linker. In addition, the fusion protein not only minimizes FcRn recycling through the IgG Fc region, but also causes mutations in the IgM Fc region and/or J chain comprised in the fusion protein, thereby blocking the possibility of reduction of the blood half-life occurring due to binding to the IgM-specific receptor, and the possibility of side effects occurring due to non-specific binding.

As used herein, the term "fusion protein" refers to a protein in which part or all of two or more different proteins are fused. The fusion protein comprises amino acid sequences of proteins constituting it, and may further comprise, but not limited to, an amino acid sequence such as a signal peptide that allows proteins produced within the cell to be secreted out of the cell.

As used herein, the term "IgG Fc region" refers to the Fc region (fragment crystallizable region) of immunoglobulin G (IgG), and also refers to the remaining portion of IgG excluding the Fab region (fragment antigen binding region). The IgG Fc region may be directly connected to the linker and the IgM Fc region in the fusion protein. In addition, the IgG Fc region may be indirectly connected to a biologically active molecule through a linker in the fusion protein, where it can function as a linker, support, or carrier for the biologically active molecule so that the fusion protein can contain one or two or more of the biologically active molecules. In this specification, the term 'IgG Fc region' may be used interchangeably with the term 'IgG Fc fragment' with the same meaning.

Specifically, the IgG Fc region monomer may be composed of two heavy chains. One heavy chain may comprise one or more selected from the group consisting of a CH2 domain and a CH3 domain. For example, one heavy chain may comprise or consist of a CH2 domain and a CH3 domain.

In the present invention, the IgG Fc region may be derived from IgG1, IgG2, IgG3, or IgG4.

Additionally, the IgG Fc region may be derived from wild-type IgG or variant IgG. Specifically, the wild-type IgG may comprise the amino acid sequence of SEQ ID NO: 1 or 3, and the variant IgG may comprise the amino acid sequence of SEQ ID NO: 2.

The variant IgG may have a mutation that reduces Antibody-Dependent Cell-mediated Cytotoxicity (ADCC) and/or a mutation that reduces Complement-Dependent Cytotoxicity (CDC). Specifically, the variant IgG may have the L14A mutation at the 14th position or the L15A mutation at the 15th position, or the L14A and L15A mutations at the 14th and 15th positions in the amino acid sequence of SEQ ID NO: 1.

As used herein, the term "IgM Fc region" refers to the Fc region (fragment crystallizable region) of immunoglobulin M (IgM) and also refers to the remaining portion of IgM excluding the Fab region (fragment antigen binding region). The IgM Fc region may be directly connected to the IgG Fc region in the fusion protein and may also be directly connected with a linker if the IgG Fc region does not exist in the fusion protein. Additionally, the IgM Fc region may be indirectly connected to the biologically active molecule protein through an IgG Fc region and a linker in the fusion protein or may be indirectly connected to the biologically active molecule only through a linker in the fusion protein. It can function as a linker, support, or carrier for the biologically active molecule so that the fusion protein can contain one or two or more of the biologically active molecules. In this specification, the term 'IgM Fc region' may be used interchangeably with the term 'IgM Fc fragment' with the same meaning.

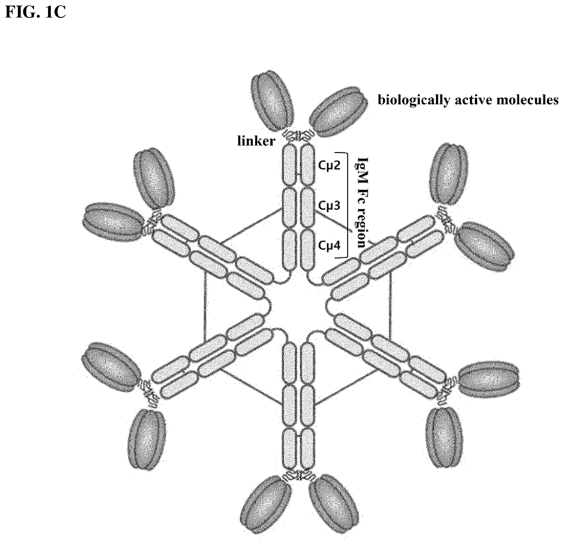

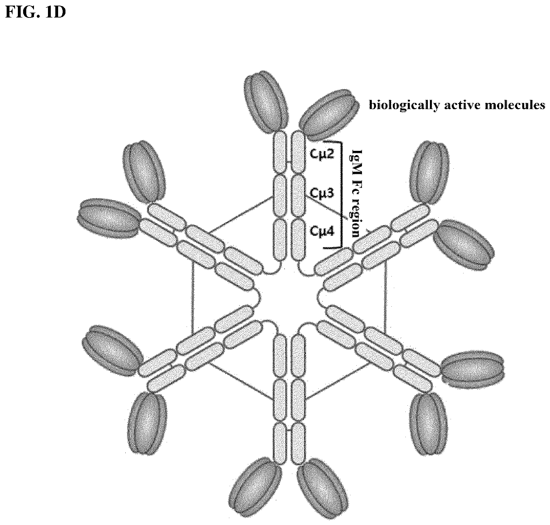

In the present invention, the IgM Fc region monomers can form a pentamer, and each monomer can be connected through a disulfide bond. The pentamer exhibits a structure containing five IgM Fc region monomers, which is shown in FIGs. 1A, 1B, 11A, and 11B. On the other hand, the IgM Fc region monomers may form a hexamer. In this case, the production yield of the fusion protein containing the hexamer is lower than that of the pentamer, and there may be a problem related to productivity, such as the generation of lots of impurities during the purification process. The hexamer exhibits a structure containing six IgM Fc region monomers, which is shown in FIGs. 1C and 1D.

Specifically, the IgM Fc region monomer may be composed of two heavy chains. One heavy chain may comprise one or more selected from the group consisting of a Cµ2 domain, a Cµ3 domain, and a Cµ4 domain.

For example, one heavy chain may comprise or be composed of a Cµ2 domain, a Cµ3 domain and a Cµ4 domain. One heavy chain may comprise or be composed of the amino acid sequence of SEQ ID NO: 4 or the nucleic acid sequence of SEQ ID NO: 5, but is not limited thereto.

As another example, one heavy chain may comprise or be composed of a Cµ3 domain and a Cµ4 domain. One heavy chain may comprise or be composed of the amino acid sequence of SEQ ID NO: 6 or the nucleic acid sequence of SEQ ID NO: 7, but is not limited thereto.

As another example, one heavy chain may comprise or be composed of a Cµ2 domain. One heavy chain may comprise or be composed of the amino acid sequence of SEQ ID NO: 8 or the nucleic acid sequence of SEQ ID NO: 9, but is not limited thereto.

As another example, one heavy chain may comprise or be composed of a Cµ3 domain. One heavy chain may comprise or be composed of the amino acid sequence of SEQ ID NO: 10 or the nucleic acid sequence of SEQ ID NO: 11, but is not limited thereto.

As another example, one heavy chain may comprise or be composed of a Cµ4 domain. One heavy chain may comprise or be composed of the amino acid sequence of SEQ ID NO: 12 or the nucleic acid sequence of SEQ ID NO: 13, but is not limited thereto.

Additionally, the IgM Fc region may be derived from wild-type IgM or variant IgM.

Specifically, the wild-type IgM may comprise or be composed of the amino acid sequence of SEQ ID NO: 4, 6, 8, 10, or 12, or the nucleic acid sequence of SEQ ID NO: 5, 7, 9, 11, or 13, but is not limited thereto.

In the variant IgM, one or more of the amino acids at the 237th and 238th positions in the amino acid sequence of SEQ ID NO: 4 may be deleted or substituted. Specifically, the variant IgM may have any one mutation selected from the group consisting of N237A, N237F and N237K at the 237th position in the amino acid sequence of SEQ ID NO: 4. In addition, the variant IgM may have any one mutation selected from the group consisting of L238A, L238D, L238E, L238F, L238G, L238H, L238I, L238K, L238M, L238N, L238P, L238Q, L238R, L238S, L238T, L238V, L238W and L238Y at the 238th position in the amino acid sequence of SEQ ID NO: 4.

In the variant IgM, one or more of the amino acids at the 124th and 125th positions in the amino acid sequence of SEQ ID NO: 6 may be deleted or substituted. Specifically, the variant IgM may have any one mutation selected from the group consisting of N124A, N124F and N124K at the 124th position in the amino acid sequence of SEQ ID NO: 6. In addition, the variant IgM may have any one mutation selected from the group consisting of L125A, L125D, L125E, L125F, L125G, L125H, L125I, L125K, L125M, L125N, L125P, L125Q, L125R, L125S, L125T, L125V, L125W and L125Y at the 125th position in the amino acid sequence of SEQ ID NO: 6.

In the variant IgM, one or more of the amino acids at the 19th and 20th positions in the amino acid sequence of SEQ ID NO: 12 may be deleted or substituted. Specifically, the variant IgM may have any one mutation selected from the group consisting of N19A, N19F and N19Kat the 19th position in the amino acid sequence of SEQ ID NO: 12.

In addition, the variant IgM may have any one mutation selected from the group consisting of L20A, L20D, L20E, L20F, L20G, L20H, L20I, L20K, L20M, L20N, L20P, L20Q, L20R, L20S, L20T, L20V, L20W and L20Y at the 20th position in the amino acid sequence of SEQ ID NO: 12.

As used herein, the term "J chain (joining chain)" is a component of an IgM or IgA antibody and refers to a polypeptide that forms a multimer by binding IgM or IgA monomers. The J chain may exist between the IgM Fc region monomers in the fusion protein and may serve to connect them. Specifically, the J chain may be comprised when the IgM Fc region monomers form a pentamer and may not be comprised when the IgM Fc region monomers form a hexamer. More specifically, when the IgM Fc region monomers form a pentamer, the J chain may bind to the first IgM Fc region monomer and the fifth IgM Fc region monomer through a disulfide bond, and such a structure is shown in FIGs. 1A, 1B, 11A, and 11B.

Additionally, the J chain may be derived from wild-type J chain or variant J chain. Specifically, the wild-type J chain may comprise or be composed of the amino acid sequence of SEQ ID NO: 14, or the nucleic acid sequence of SEQ ID NO: 15, but is not limited thereto.

In the variant J chain, one or more of the amino acids at the 105th, 106th and 107th positions in the amino acid sequence of SEQ ID NO: 14 may be deleted or substituted. Specifically, the variant J chain may have any one mutation selected from the group consisting of D105A and D105K at the 105th position in the amino acid sequence of SEQ ID NO: 14. Specifically, the variant J chain may have any one mutation selected from the group consisting of R106A, R106D, R106E, R106F, R106G, R106H, R106I, R106K, R106L, R106M, R106N, R106P, R106Q, R106S, R106T, R106V, R106W and R106Y at the 106th position in the amino acid sequence of SEQ ID NO: 14. In addition, the variant J chain may have any one mutation selected from the group consisting of N107A, N107E, N107F and N107K at the 107th position in the amino acid sequence of SEQ ID NO: 14.

In the present invention, the J chain may be one to which a protein molecule is additionally bound. The protein molecule may exhibit a synergistic effect on the desired effect of the fusion protein of the present invention. Specifically, it may be, but not limited to, one or more selected from the group consisting of antibodies, antigen-binding fragments of antibody, antibody-drug conjugates, antibody-like molecules, antigen-binding fragments of antibody-like molecule, soluble proteins, membrane-bound proteins, ligands, receptors, virus-like particles, protein toxins and enzymes. More specifically, it may be one or more selected from the group consisting of a co-stimulatory receptors, cytokines, and cell engagers. The cell engager may be an antibody, in particular, an antibody that binds to a cancer cell or immune cell-specific targeting protein, for example, a T-cell engager. In addition, the immune cell may include neutrophils, eosinophils, basophils, mast cells, monocytes, macrophages, dendritic cells, natural killer cells (NK cells), and lymphocytes such as B cells and T cells.

As used herein, the term "biologically active molecule" refers to a substance capable of exhibiting a particular action or effect in an organism. For example, it refers to a substance capable of promoting or inhibiting the expression of a particular gene, enhancing, or inhibiting the function of a particular protein, or inducing or inhibiting the cell death, by activating or inactivating a particular signaling pathway. Preferably, it may exhibit immune regulations and/or anticancer activities.

In the present invention, the biologically active molecule may be indirectly linked to the IgG Fc region through a linker in the fusion protein, or the biologically active molecule may be indirectly linked to the IgM Fc region through a linker in the fusion protein. Here, it may serve as a functional group or active moiety that can exhibit the desired effect of the fusion protein.

Specifically, it may be, but not limited to, one or more selected from the group consisting of antibodies, antigen-binding fragments of antibody, antibody-drug conjugates, antibody-like molecules, antigen-binding fragments of antibody-like molecule, soluble proteins, membrane-bound proteins, ligands, receptors, virus-like particles, protein toxins, chemokines, cytokines, and enzymes.

The receptor may be, but not limited to, a cytokine receptor and/or an immune checkpoint receptor. For example, the fusion protein according to the present invention may comprise two or more kinds of biologically active molecules depending on a desired purpose, target, or effect.

For example, the antigen-binding fragment of antibody may be, but not limited to, at least one selected from the group consisting of Fab, F(ab')₂, F(ab)₂, Fab', Fab₂, Fab₃, Fv, scFv, Bis-scFv, minibody, triabody, diabody, tandem diabody (TandAb), nanobody and tetrabody. In addition, it may be one that binds to HLA-G. The HLA-G, which belongs to HLA class I, is expressed in the placenta of pregnant women, and is involved in the immune tolerance of fetus. It binds to ILT2, ILT4 or KIR2DL4, which is an inhibitory receptor of natural killer (NK) cell, to inhibit NK cells, and promotes the differentiation of regulatory T cells (T_{reg}). In addition, when a cancer cell overexpresses HLA-G on the surface, it inhibits the activities of helper T-cells, NK cells, and cytotoxic T-cells involved in cytotoxicity, thereby suppressing the immunotherapeutic effect for cancer treatment. Therefore, an antigen-binding fragment that selectively binds to HLA-G and inhibits the binding of HLA-G to an inhibitory receptor may be used as an anticancer therapeutic substance. For example, the antigen-binding fragment of antibody may be a Fab or scFv. The light chain (VL domain and CL domain) constituting the Fab may comprise or consist of the amino acid sequence of SEQ ID NO: 26 or the nucleic acid sequence of SEQ ID NO: 27, but is not limited thereto. The part of the heavy chain (VH domain and CH1 domain) may comprise or consist of the amino acid sequence of SEQ ID NO: 30 or the nucleic acid sequence of SEQ ID NO: 31, but is not limited thereto.

In another example, the receptor may be Human Leukocyte Antigen (HLA), but is not limited thereto. The term, 'HLA' refers to a protein encoded by a human major histocompatibility complex (MHC) gene complex and is an important immunological molecule which is mainly present in a form binding to a membrane and is responsible for the regulation of immune system. HLA activates cytotoxic T-cells by presenting a peptide fragment of foreign or autologous protein on the cell surface and allowing the T Cell Receptor (TCR) of the cytotoxic T-cell to recognize and bind it. HLA may be mainly divided into two types. HLA class I is present on the surface of almost all cells, whereas HLA class II presents only in an particular antigen presenting cell (APC) such as NK cells, macrophages and dendritic cells. The HLA gene is highly polymorphic and expresses six different HLA protein α chains (two HLA-A, two HLA-B and two HLA-C). The HLA class I can selectively activate cytotoxic T-cells expressing TCR when a specific peptide fragment is loaded thereto, and thus the recombinant HLA class I in which the membrane-bound domain has been removed may be used for the treatment of cancer or infectious disease. Unlike HLA class II, most of the HLA class I is unstable when the peptide is not loaded in the groove. Therefore, recombinant HLA class I is prepared in a form loading the target synthetic peptide or in a form fused to the α chain. In addition, since there is a risk that refolding may not occur properly when expressed using a microorganism, it is general to express using animal cells. In the art, HLA and MHC are used interchangeably with the same meaning, and HLA is used instead of MHC in human. In this specification, the terms 'HLA' and 'MHC' may be used interchangeably with the same meaning.

The HLA may be HLA class I such as HLA-A, HLA-B, HLA-C, HLA-E, HLA-F and HLA-G; or HLA class II such as HLA-DP, HLA-DM, HLA-DOA, HLA-DOB, HLA-DQ and HLA-DR. The HLA class I consists of a β2m domain, an α1 domain, an α2 domain and an α3 domain, and the HLA class II consists of an α1 domain, an α2 domain, a β1 domain and a β2 domain. The HLA of the present invention may comprise a β2m domain, an α1 domain, an α2 domain or an α3 domain, or an α1 domain, an α2 domain, a β1 domain and a β2 domain. The α2 domain may have a substitution from Gln (glutamine; Q) to Glu (glutamic acid; E) at the 115th position. The α1 domain may have a substitution from Tyr (tyrosine; Y) to Cys (cysteine; C) at the 84th position. When Gln at the 115th position of the α2 domain is substituted with Glu, the binding force of HLA with TCR present in CD8⁺ T-cells is increased by about 1.5 times compared to that not substituted. When Tyr at the 84th position of the α1 domain is substituted with Cys, HLA forms a disulfide bond with the peptide bound thereto, and thus the stability of HLA can be improved.

In addition, the HLA may comprise a substance that specifically binds to the active site of the target T-cell. The substance may selectively activate TCR-expressing target T-cells, and/or increase the stability of HLA. The term, 'target T-cell' may be a T-cell to which the fusion protein of the present invention binds or may be a cytotoxic T cell (killer T cell; Tc) or a helper T cell (Th). The cytotoxic T-cell is a cell capable of killing cancer cells, cells infected with viruses, or damaged cells, and may be, for example, CD8⁺ T-cells. CD8, a protein receptor of CD8⁺ T-cells, is located on the cell surface and binds to HLA class I. The helper T-cell does not directly kill cells but play a role in activating other immune cells. For example, it promotes an antibody production of B-cell and an activation of cytotoxic T-cell and may be CD4⁺ T-cells. CD4, a protein receptor of CD4⁺ T-cells, is located on the cell surface and binds to HLA class II.

An example of the substance that specifically binds to the active site of the target T-cell may be a peptide, but is not limited thereto. The peptide may be derived from a virus or an animal cell. The virus may be a cytomegalovirus (CMV), but is not limited thereto. CMV is one of the most potent immunogenic antigens for the human immune system and stimulates a very strong CD8⁺ T-cell response upon infection in human. The CD8⁺ T-cell immune response is primarily activated by CMV proteins pp65 and IE-1, and it is known that CMV-specific T-cells for each peptide is very high, up to about 1% to 2% of the total CD8⁺ T-cell repertoire. For example, the HLA comprising CMV may comprise or be composed of the amino acid sequence of SEQ ID NO: 22 or 24, or the nucleic acid sequence of SEQ ID NO: 23 or 25, but is not limited thereto.

In another example, the ligand may be EPO (erythropoietin) or an EPO analog, but is not limited thereto. The term 'EPO' is a hormone protein essential for red blood cell production, which acts on hematopoietic cells in bone marrow to promote the differentiation and growth of red blood cells, thereby treating anemia, protecting nerves in hypoxic environments such as stroke, and involving in the wound repair process. In addition, it is known to be effective in the treatment of depression and improve memory by involving in the hippocampal response, synaptic connection, and neuronal network in the brain. The 'EPO analog' is a recombinant protein that performs the same function as EPO and has a different structure. It has two additional sugar chains in addition to the three sugar chains of human EPO and has a half-life in blood that is three times or more improved compared to the existing human EPO. It may be a ligand, antibody, or antibody fragment capable of binding to and activating an EPO receptor, and may be, but not limited to, Novel Erythropoiesis Stimulating Protein (NESP). For example, it may comprise or be composed of the amino acid sequence of SEQ ID NO: 32, or the nucleic acid sequence of SEQ ID NO: 33, but is not limited thereto.

As used herein, the term 'linker' refers to an amino acid present between each domain of a protein. The linker may exist between the biologically active molecule and the IgG Fc region in the fusion protein and may serve to link them. In addition, it may exist between the biologically active molecule and the IgM Fc region when the IgG Fc region does not exist in the fusion protein and may serve to connect the biologically active molecule and the IgM Fc region.

As a specific example, the linker may be at least one selected from the group consisting of an IgD hinge region, an IgA hinge region, an IgG hinge region, and a GS linker.

The 'hinge region' refers to a part located in the central portion of the heavy chain of an antibody and connecting two regions of the heavy chain by a disulfide bond. It is located between the CH1 domain and the CH2 domain of the heavy chain and is connected to them. An immunoglobulin having a short hinge part has a Y-shape, whereas IgD having a long hinge part may have great flexibility such that both target binding sites spread apart to form a T-shape. This flexibility enables IgD to have a binding activity capable of binding to a multimeric foreign antigen with little binding to a self-antigen. In this specification, 'hinge region' may be used interchangeably with 'hinge domain' with the same meaning.

In the present invention, the hinge region may comprise a part or all of hinge region, a part or all of CH1 domain, a part or all of CH2 domain, or a combination thereof.

For example, the hinge region may comprise a part or all of IgD hinge (hereinafter, 'part or all' is indicated as 'part/all'). Specifically, it may comprise a part/all of IgD hinge and a part/all of IgD CH1 domain. Specifically, it may comprise a part/all of IgD hinge and a part/all of IgD CH2 domain. Specifically, it may comprise a part/all of IgD hinge, a part/all of IgD CH1 domain, and a part/all of IgD CH2 domain.

As another example, the hinge region may comprise a part/all of IgA hinge. Specifically, it may comprise a part/all of IgA hinge and a part/all of IgA CH1 domain. Specifically, it may comprise a part/all of IgA hinge and a part/all of IgA CH2 domain. Specifically, it may comprise a part/all of IgA hinge, a part/all of IgA CH1 domain, and a part/all of IgA CH2 domain.

As another example, the hinge region may comprise a part/all of IgG hinge. Specifically, it may comprise a part/all of IgG hinge and a part/all of IgG CH1 domain. Specifically, it may comprise a part/all of IgG hinge and a part/all of IgG CH2 domain. Specifically, it may comprise a part/all of IgG hinge, a part/all of IgG CH1 domain, and a part/all of IgG CH2 domain.

Specifically, the IgD hinge region may comprise or be composed of the amino acid sequence of SEQ ID NO: 16 or the nucleic acid sequence of SEQ ID NO: 17, but is not limited thereto. In addition, the IgA hinge region may comprise or be composed of the amino acid sequence of SEQ ID NO: 18 or the nucleic acid sequence of SEQ ID NO: 19, but is not limited thereto.

The `GS linker' may be at least one selected from the group consisting of (GS)_{d}, (SG)_{d}, (GGGS)_{d}, (GGGGS)_{d}, GCGS(GGGS)_{d} and GCGGS(GGGGS)_{d}, wherein the d is an integer from 2 to 6. Specifically, it may comprise 4 or more and less than 35 amino acids consisting of glycine (Gly; G) and serine (Ser; S). Otherwise, it may comprise 4 or more and less than 35 amino acids consisting of glycine, serine and cysteine (Cys; C) in order to increase stability through a disulfide bond, but is not limited thereto. In addition, the GS linker may comprise or be composed of the amino acid sequence of SEQ ID NO: 20 or the nucleic acid sequence of SEQ ID NO: 21.

Another aspect of the present invention provides a nucleic acid molecule encoding the fusion protein.

In the nucleic acid molecule according to the present invention, each term is understood to have the same meaning as described previously, unless otherwise specified.

As used herein, the term `nucleic acid molecule' has a meaning comprehensively including DNA and RNA molecules. Nucleotides, which are the basic structural units in the nucleic acid molecule, include not only natural nucleotides but also analogues in which sugar or base sites are modified. The sequence of the nucleic acid molecule encoding the fusion protein of the present invention may be modified, and such a modification includes addition, deletion, or non-conservative or conservative substitution of nucleotides.

In addition, all sequences used in the present invention, including nucleic acid sequences and amino acid sequences, are construed to include sequences that exhibit substantial identity to the sequences described in the sequence list, considering mutations with biologically equivalent activity. The term, 'substantial identity' means a sequence showing at least 60% homology, more specifically 70% homology, even more specifically 80% homology, most specifically 90%, 91%, 92%, 93%, 94%, 95%, 96%, 97%, 98% or 99% homology when the sequence of the present invention and any other sequences are aligned to the maximum extent, and the aligned sequences are analyzed using an algorithm commonly used in the art.

Accordingly, sequences having high homology with the sequences represented by SEQ ID NOs: 1 to 33 of the present invention, for example, having at least 70%, specifically at least 80%, more specifically at least 90% homology should also be construed as being included in the scope of the present invention.

Another aspect of the present invention provides a vector for expression of a fusion protein comprising the nucleic acid molecule.

In the vector for expression of a fusion protein according to the present invention, each term is understood to have the same meaning as described previously, unless otherwise specified.

As used herein, the term 'vector' refers to a plasmid, a virus or any other carrier known in the art, which can insert or introduce a nucleic acid molecule encoding the fusion protein into a host cell, as a means for expressing the fusion protein of the present invention. The vector may be constructed as a vector for cloning or a vector for expression. Specific examples may be viral vectors such as plasmid vectors, cosmid vectors, bacteriophage vectors, adenoviral vectors, retroviral vectors and adeno-associated viral vectors, and more specifically, plasmids (e.g., pcDNA series such as pcDNA3 or pcDNA3.1, pCL, pSC101, pGV1106, pACYC177, ColE1, pKT230, pME290, pBR322, pUC8/9, pUC6, pBD9, pHC79, pU61, pLAFR1, pHV14, pGEX series, pET series and pUC19), phages (e.g., λgt4·λB, λ-Charon, λΔz1, and M13) or viruses (e.g., SV40), but are not limited thereto.

The vector of the present invention may be one in which the nucleic acid molecule encoding the fusion protein is operably linked to a promoter. As used herein, the term "operably linked" refers to a functional linkage between a nucleic acid expression control sequence (e.g., promoter, signal sequence, or array of transcription factor binding sites) and other nucleic acid sequences, whereby the control sequence regulates the transcription and/or translation of the other nucleic acid sequences.

When the vector of the present invention is an expression vector and a eukaryotic cell is a host, a promoter derived from the genome of mammalian cell (e.g., metallothionein promoter, β-actin promoter, human hemoglobin promoter, human muscle creatine promoter) or promoters derived from viruses (e.g., adenovirus late promoter, vaccinia virus 7.5K promoter, SV40 promoter, cytomegalovirus (CMV) promoter, tk promoter of HSV, mouse mammary tumor virus (MMTV) promoter, HIV LTR promoter, promoter of Moloney virus, promoter of Epstein Barr virus (EBV), promoter of Rous sarcoma virus (RSV)) may be used, and a polyadenylation sequence may be comprised as a transcription termination sequence. For example, the recombinant vector of the present invention may comprise a CMV promoter, but is not limited thereto.

When the vector of the present invention is an expression vector and a prokaryotic cell is used as a host, a strong promoter capable of driving transcription (e.g., tac promoter, lac promoter, lacUV5 promoter, lpp promoter, pLλ promoter, pRλ promoter, rac5 promoter, amp promoter, recA promoter, SP6 promoter, trp promoter, T7 promoter), a ribosome binding site for start of translation, and a transcription/translation termination sequence can be used. For example, when *Escherichia coli* (e.g., HB101, BL21, DH5α, etc.) is used as the host cell, a promoter and operator site of *E. coli* tryptophan biosynthesis pathway, or a left-side promoter of phage λ (pLλ promoter) may be used as a regulatory site. When *Bacillus* bacteria is used as a host cell, a promoter of toxin protein gene of *Bacillus thuringiensis* or any promoter that can be expressed in *Bacillus* bacteria may be used as a regulatory site.

The recombinant vector system of the present invention may be constructed through various methods known in the art and may comprise antibiotic resistance genes commonly used in the art as selection markers (e.g., ampicillin, gentamicin, carbenicillin, chloramphenicol, streptomycin, kanamycin, geneticin, neomycin or tetracycline resistance genes).

Another aspect of the present invention provides a host cell into which the vector for expression of the fusion protein is introduced.

In the host cell according to the present invention, each term is understood to have the same meaning as described previously, unless otherwise specified.

As used herein, the term `host cell' refers to a cell that contains the vector for expression of the fusion protein and can stably and continuously clone and express the fusion protein of the present invention. For example, it may be, but not limited to, prokaryotic host cells such as *Bacillus* genus strains including *Escherichia coli, Bacillus subtilis* and *Bacillus thuringiensis, Streptomyces, Pseudomonas, Proteus mirabilis* or *Staphylococcus;* eukaryotic host cells such as fungi including *Aspergillus* species, *Pichia pastoris, Saccharomyces cerevisiae, Schizosaccharomyces* or *Neurospora crassa;* lower eukaryotic cells; higher eukaryotic cells such as insect-derived cells; plant cells; or mammal-derived cells such as COS7 cells (monkey kidney cells), NSO cells, SP2/0, Chinese hamster ovary (CHO) cells, W138, baby hamster kidney (BHK) cells, MDCK, myeloma cell line, HuT 78 cells or 293 cells. The host cells commonly used in the art may be used without limitation.

Another aspect of the present invention provides a method for preparing the fusion protein, comprising the step of introducing the vector for expression of the fusion protein into the host cell.

In the method for preparing the fusion protein according to the present invention, each term is understood to have the same meaning as described previously, unless otherwise specified.

Specifically, the method for preparing the fusion protein of the present invention may comprise:
(a) introducing the vector of the present invention into a host cell;
(b) culturing the host cell; and
(c) obtaining a fusion protein from the host cell.

The step (a) of introducing the vector of the present invention into a host cell may be a step of preparing a transformant comprising the vector for expression of the fusion protein. The step of preparing the transformant may be performed by transforming the host cell.

As used herein, the term 'transformant' refers to an organism in which genetic changes are artificially made by introducing foreign DNA into a cell, where DNA is introduced into a host and becomes replicable as a factor of chromosomes or by the chromosomal integration.

Transformation of the host cells may be performed using any transformation method, and be easily performed according to methods conventionally used in the art. For example, it may comprise, but not limited to, CaCl₂ precipitation method, Hanahan method in which dimethyl sulfoxide (DMSO) is used in CaCl₂ method to increase the efficiency, electroporation, calcium phosphate precipitation method, protoplast fusion method, agitation using silicon carbide fibers, agrobacterium mediated transformation method, transformation method using PEG, dextran sulfate, lipofectamine or drying/inhibition mediated transformation method, etc. Transformation or transfection methods commonly used in the art may be used without limitation.

The step (b) of culturing the host cell in the present invention may be performed using a medium and culture condition known in the art. Such a culture may be easily adjusted and used by those skilled in the art depending on the strain selected. Suspension culture or adherent culture may be used depending on the cell growth type; or batch, fed-batch or continuous culture methods may be used depending on the culture type.

In the culture for animal cells, the medium may contain various carbon sources, nitrogen sources and trace element components. Examples of the carbon source may include carbohydrates such as glucose, sucrose, lactose, fructose, maltose, starch, or cellulose; fats such as soybean oil, sunflower oil, castor oil or coconut oil; fatty acids such as palmitic acid, stearic acid, or linoleic acid; alcohols such as glycerol or ethanol; or an organic acid such as acetic acid. These carbon sources may be used alone or in combination. Examples of the nitrogen source may include, for example, organic nitrogen sources such as peptone, yeast extract, broth, malt extract, corn liquor or soybean wheat; or an inorganic nitrogen source such as urea, ammonium sulfate, ammonium chloride, ammonium phosphate, ammonium carbonate or ammonium nitrate. These nitrogen sources may be used alone or in combination. Examples of the trace components may include phosphorus sources such as potassium dihydrogen phosphate or dipotassium hydrogen phosphate; or metal salts such as magnesium sulfate or iron sulfate. In addition, amino acids, vitamins, or suitable precursors may be comprised.

In addition, compounds such as ammonium hydroxide, potassium hydroxide, ammonia, phosphoric acid, or sulfuric acid may be added to the medium in an appropriate manner in order to adjust the pH of the culture. In order to suppress bubble formation, an antifoaming agent such as fatty acid polyglycol ester may be used during culture. Oxygen or oxygen-containing gas (e.g., air) can be injected into the culture in order to maintain the aerobic state of the culture.

In addition, it may be cultured while maintaining the temperature of the culture at 20 °C to 45 °C, specifically 25 °C to 40 °C.

The step (c) of expressing the fusion protein in the host cell according to the present invention may be a step of recovering, purifying, and concentrating the fusion protein of the present invention from the culture solution obtained through the step (b).

The fusion protein may be used in an unpurified state, or after additional recovery, purification and concentration. Specifically, methods commonly used in the art (e.g., dialysis, salt precipitation, chromatography) may be used, through which recovery, purification and concentration may be simultaneously performed. More specifically, it may be recovered, purified and concentrated using chromatography (e.g., ion exchange chromatography, size exclusion chromatography, or affinity chromatography), and the type and order of the column used for this may be appropriately selected depending on the characteristics of the fusion protein or culture method.

Another aspect of the present invention provides a use of the fusion protein for preparing pharmaceuticals for treatment, prevention, or diagnosis.

Another aspect of the present invention provides a pharmaceutical composition comprising the fusion protein.

Another aspect of the present invention provides a method for treating or preventing a disease in a subject, comprising administering the fusion protein to the subject.

In the use, pharmaceutical composition, and method according to the present invention, each term is understood to have the same meaning as described previously, unless otherwise specified.

If the fusion protein comprises a biologically active molecule that exhibits an effect such as immunoregulation or anticancer, it may be used in a subject in need of treatment, prevention, improvement, or diagnosis of diseases caused by a decrease or loss of immunoregulatory function or cancer.

The 'subject' may include humans, any non-human animals, fish, or plants, etc., without limitation. The non-human animal may be a vertebrate, for example primate, dog, cow, horse, pig, rodent, for example mouse, rat, hamster, guinea pig, etc. In this specification, the "subject" may be used interchangeably with 'individual' or 'patient'.

In one embodiment according to the present invention, the biologically active molecule included in the fusion protein may exhibit immunomodulatory activity, anticancer activity, or anti-inflammatory activity, but is not limited thereto. In addition, the biologically active molecule may comprise or consist of the amino acid sequence of SEQ ID NO: 22, 24, 26, 28, 30 or 32, or the nucleic acid sequence of SEQ ID NO: 23, 25, 27, 29, 31 or 33, but is not limited thereto. A person skilled in the art will be able to appropriately select a biologically active molecule depending on the subject's disease and its severity or condition.

In this specification, 'A, B and/or C' is used to mean A, or B, or C, or A and B, or A and C, or B and C, or A, B and C.

### [Brief Description of Figures]

FIG. 1A is a schematic diagram of a fusion protein according to the present invention. The fusion protein comprises the biologically active molecules, the linker, the IgM Fc regions, and the J chain. The IgM Fc and the J chain form a pentamer.
FIG. 1B is a schematic diagram of a fusion protein according to the present invention. The fusion protein comprises the biologically active molecules, the IgM Fc regions, and the J chain, but does not comprise the linker. The IgM Fc form a pentamer.
FIG. 1C is a schematic diagram of a fusion protein according to the present invention. The fusion protein comprises the biologically active molecules, the linker, and the IgM Fc regions, but does not comprise the J chain. The IgM Fc form a hexamer.
FIG. 1D is a schematic diagram of a fusion protein according to the present invention. The fusion protein comprises the biologically active molecules and the IgM Fc regions, but does not comprise the linker and the J chain. The IgM Fc form a pentamer.

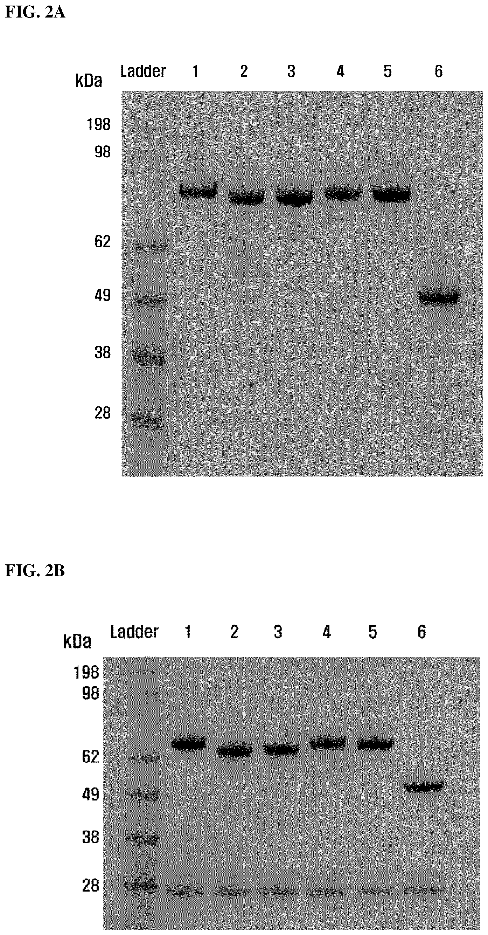

FIG. 2A is an image showing the results of reducing SDS-PAGE analysis of the fusion protein comprising HLA (CMVpHLA) as a biologically active molecule. 'Ladder' represents a protein size marker; '1' represents the fusion protein with the structure of [(CMVpHLA)₂-(IgD hinge region)₂-(IgM Fc region)]s-(J chain); '2' represents the fusion protein with the structure of [(CMVpHLA)₂-(linker)₀-(IgM Fc region)]s-(J chain); '3' represents the fusion protein with the structure of [(CMVpHLA)₂-((GGGGS)₃)₂-(IgM Fc region)]s-(J chain); '4' represents the fusion protein with the structure of [(CMVpHLA)₂-(IgA hinge region)₂-(IgM Fc region)]s-(J chain); '5' represents the fusion protein with the structure of [(CMVpHLA)₂-(IgD hinge region)₂-(IgM Fc region)]₆-(J chain)₀; '6' represents the control CMVpHLA protein.
FIG. 2B is an image showing the results of reducing SDS-PAGE analysis of the fusion protein comprising antibody fragment (IgG Fab; A2014) as a biologically active molecule. 'Ladder' represents a protein size marker; '1' represents the fusion protein with the structure of [(A2014)₂-(IgD hinge region)₂-(IgM Fc region)]s-(J chain); '2' represents the fusion protein with the structure of [(A2014)₂-(linker)₀-(IgM Fc region)]s-(J chain); '3' represents the fusion protein with the structure of [(A2014)₂-((GGGGS)₃)₂-(IgM Fc region)]s-(J chain); '4' represents the fusion protein with the structure of [(A2014)₂-(IgA hinge region)₂-(IgM Fc region)]s-(J chain); '5' represents the fusion protein with the structure of [(A2014)₂-(IgD hinge region)₂-(IgM Fc region)]₆-(J chain)₀; '6' represents the control (A2014)-(IgG Fc) fusion protein.
FIG. 2C is an image showing the results of reducing SDS-PAGE analysis of the fusion protein comprising EPO analogue as a biologically active molecule. 'Ladder' represents a protein size marker; '1' represents the fusion protein with the structure of [(NESP)₂-(IgD hinge region)₂-(IgM Fc region)]s-(J chain); '2' represents the fusion protein with the structure of [(NESP)₂-(linker)₀-(IgM Fc region)]s-(J chain); '3' represents the control NESP.
FIG. 3A is a graph showing the ELISA results of the target binding capacity of the fusion protein comprising HLA (CMVpHLA) as a biologically active molecule.
FIG. 3B is a graph showing the ELISA results of the target binding capacity of the fusion protein comprising antibody fragment (IgG Fab; A2014) as a biologically active molecule.
FIG. 3C is a graph showing the ELISA results of the target binding capacity of the fusion protein comprising EPO analogue as a biologically active molecule.
FIG. 4 is a graph showing the results of ELISPOT analysis analyzing the amount of IFN-γ secreted by the fusion protein according to the present invention, showing that the fusion protein can induce activation of CD8+ T cells.
FIG. 5A is a graph showing the results of FACS analyzing the degree of CD8⁺ T cells proliferation by the fusion protein according to the present invention, which relates to [(CMVpHLA)₂-(linker)₀-(IgM Fc region)]s-(J chain). It shows that the fusion protein can induce proliferation of CD8+ T cells.
FIG. 5B is a graph showing the results of FACS analyzing the degree of CD8⁺ T cells proliferation by the fusion protein according to the present invention, which relates to [(CMVpHLA)₂-(IgD hinge region)₂-(IgM Fc region)]s-(J chain). It shows that the fusion protein can induce proliferation of CD8+ T cells.
FIG. 5C is a graph showing the results of FACS analyzing the degree of CD8⁺ T cells proliferation by the fusion protein according to the present invention, which relates to [(CMVpHLA)₂-(IgD hinge region)₂-(IgM Fc region)]s-(J chain). It shows that the fusion protein can induce proliferation of CD8+ T cells.
FIG. 5D is a graph showing the results of FACS analyzing the degree of CD8⁺ T cells proliferation by the fusion protein according to the present invention, which relates to [(CMVpHLA)₂-((GGGGS)₃)₂-(IgM Fc region)]s-(J chain). It shows that the fusion protein can induce proliferation of CD8+ T cells.
FIG. 5E is a graph showing the results of FACS analyzing the degree of CD8⁺ T cells proliferation by the fusion protein according to the present invention, which relates to [(CMVpHLA)₂-(IgA hinge region)₂-(IgM Fc region)]s-(J chain). It shows that the fusion protein can induce proliferation of CD8+ T cells.
FIG. 5F is a graph showing the results of FACS analyzing the degree of CD8⁺ T cells proliferation by the fusion protein according to the present invention, which relates to [(CMVpHLA)₂-(IgD hinge region)₂-(IgM Fc region)]₆-(J chain)₀. It shows that the fusion protein can induce proliferation of CD8+ T cells.
FIG. 5G is a graph showing the results of FACS analyzing the degree of CD8⁺ T cells proliferation by the fusion protein according to the present invention, which relates to the control (CMVpHLA).
FIG. 6 is a graph showing the results of FACS analyzing the cytotoxicity by the fusion protein according to the present invention, showing that the fusion protein can induce killing cancer cells by activating CD8+ T cells.
FIG. 7 is a graph showing the results of an assay analyzing the binding capacity of the fusion protein according to the present invention to HLA-G expressing cells, showing that the fusion protein can bind to the HLA-G receptor in the cell.
FIG. 8 is a graph showing the results of an assay analyzing the degree of binding the recombinant HLA-G protein and the recombinant ILT-2 protein by the fusion protein according to the present invention, showing that the fusion protein can block the binding HLA-G and ILT-2 protein.
FIG. 9 is a graph showing the results of FACS analyzing the degree of binding the recombinant HLA-G protein and the ILT-2 protein in the cell by the fusion protein according to the present invention, showing that the fusion protein can block the binding HLA-G and ILT-2 receptor protein in the cell.
FIG. 10 is a graph showing the results of an assay analyzing the proliferative ability of EPO-expressing cells by the fusion protein according to the present invention, showing that the fusion protein can bind to the EPO receptor expressed in the cell and promote the proliferation of cells expressing it.
FIG. 11A is a schematic diagram of a fusion protein according to the present invention. The fusion protein comprises the biologically active molecules, the linker, the IgM Fc regions, and the J chain. The IgM Fc and the J chain form a pentamer. The IgM Fc region and J chain is a form comprising amino acid substitutions and deletions.
FIG. 11B is a schematic diagram of a fusion protein according to the present invention. The fusion protein comprises the biologically active molecules, the linker, the IgG Fc region, the IgG Fc region, the IgM Fc regions, and the J chain. The IgM Fc and the J chain form a pentamer. The IgM Fc region and J chain is a form comprising amino acid substitutions and deletions.

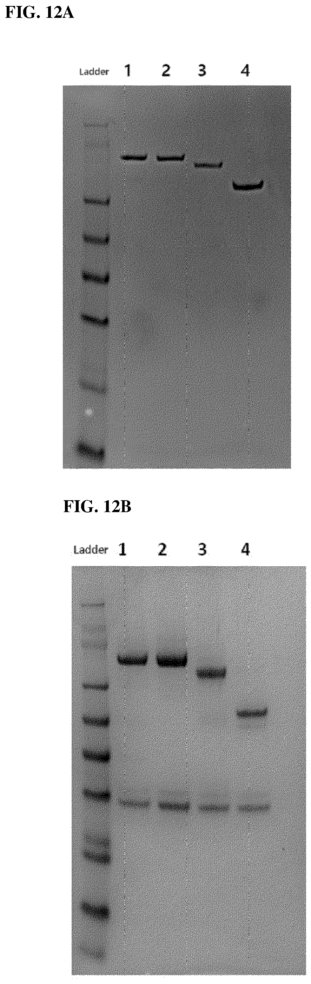

FIG. 12A is an image showing the results of reducing SDS-PAGE analysis of the fusion protein comprising CMVpHLA as a biologically active molecule. 'Ladder' represents a protein size marker; '1' represents the pentamer fusion protein with the structure of [(CMVpHLA)₂-(linker)₂-(IgG Fc region)-(IgM Fc region comprising amino acid mutations)]s-(J chain comprising amino acid mutations); '2' represents the pentamer fusion protein with the structure of [(CMVpHLA)₂-(linker)₂-(IgG Fc region)-(IgM Fc region)]s-(J chain); '3' represents the pentamer fusion protein with the structure of [(CMVpHLA)₂-(linker)₂-(IgM Fc region)]s-(J chain); '4' represents the fusion protein with the structure of (CMVpHLA)₂-(IgG Fc region).
FIG. 12B is an image showing the results of reducing SDS-PAGE analysis of the fusion protein comprising antibody fragment (A2014) as a biologically active molecule. 'Ladder' represents a protein size marker; '1' represents the pentamer fusion protein with the structure of [(A2014)₂-(linker)₂-(IgG Fc region)-(IgM Fc region comprising amino acid mutations)]s-(J chain comprising amino acid mutations); '2' represents the pentamer fusion protein with the structure of [(A2014)₂-(linker)₂-(IgG Fc region)-(IgM Fc region)]s-(J chain); '3' represents the pentamer fusion protein with the structure of [(A2014)₂-(linker)₂-(IgM Fc region)]s-(J chain); '4' represents the fusion protein with the structure of (A2014)₂-(IgG Fc region).
FIG. 13A is a graph showing the results of FACS analyzing the degree of CD8⁺ T cells proliferation by the fusion protein comprising HLA as a biologically active molecule according to the present invention.
FIG. 13B is a graph showing the results of ELISA analyzing the ILT-2 blocking ability by the fusion protein comprising antibody fragment (A2014) as a biologically active molecule according to the present invention.
FIG. 14A is a graph showing the results of ELISA measuring the affinity of the fusion protein according to the present invention with FcRn, showing the results of analysis of IMB-4000, IMB-4001, IMB-4011, and IMB-4012 at pH 6.0. It shows that the FcRn recycling effect of the fusion protein can be maximized.
FIG. 14B is a graph showing the results of ELISA measuring the affinity of the fusion protein according to the present invention with FcRn, showing the results of analysis of IMB-4000, IMB-4001, IMB-4011, and IMB-4012 at pH 7.4. It shows that the FcRn recycling effect of the fusion protein can be maximized.
FIG. 14C is a graph showing the results of ELISA measuring the affinity of the fusion protein according to the present invention with FcRn, showing the results of analysis of A2014-1, A2014-2, A2014-3 and A2014-4 at pH 6.0. It shows that the FcRn recycling effect of the fusion protein can be maximized.
FIG. 14D is a graph showing the results of ELISA measuring the affinity of the fusion protein according to the present invention with FcRn, showing the results of analysis of A2014-1, A2014-2, A2014-3 and A2014-4 at pH 7.4. It shows that the FcRn recycling effect of the fusion protein can be maximized.
FIG. 15 is a graph showing the results of ELISA measuring the affinity of the fusion protein according to the present invention with pIg receptor(pIgR). It shows that the half-life in the blood of the fusion protein can be improved.
FIG. 16 is a graph showing the results of ELISA measuring the affinity of the fusion protein according to the present invention with Fcα/µ receptor. It shows that the non-specific binding of the fusion protein can be reduced and the half-life in the blood of the fusion protein can be improved.
FIG. 17 is a graph showing the results of ELISA measuring the affinity of the fusion protein according to the present invention with Fcµ receptor. It shows that the non-specific binding of the fusion protein can be reduced and the half-life in the blood of the fusion protein can be improved.
FIG. 18 is a graph showing the results of a blood half-life measurement of the fusion protein according to the present invention. The fusion protein exhibits the effect of maximizing half-life in the blood through inhibition of FcRn recycling and inhibition of IgM-specific receptor binding.

### [EXAMPLES]

Hereinafter, the present invention will be described in more detail by examples. However, these examples are for illustrative purposes only, and the scope of the present invention is not limited by these examples.

### Example 1. Preparation of fusion protein platform comprising IgM Fc region

In Example 1, a fusion protein platform comprising a biologically active molecule, linker, IgM Fc region and/or J chain was prepared.

### Example 1-1. Preparation of fusion protein comprising IgM Fc region, J chain and linker

In Example 1-1, a recombinant plasmid comprising IgM Fc region, J chain and/or linker was prepared. Cµ2 domain, Cµ3 domain and Cµ4 domain of wild-type IgM were used as the IgM Fc region.

Specifically, the inventors constructed a recombinant plasmid for expressing the IgM Fc region (SEQ ID NO: 5) comprising the Cµ2 domain, the Cµ3 domain and the Cµ4 domain and the J chain (SEQ ID NO: 15) required for pentamer formation. In addition, in order to solve the steric hindrance problem of existing IgM antibodies, we constructed a recombinant plasmid for the fusion protein in which a human IgD hinge region (SEQ ID NO: 17), a human IgA hinge region (SEQ ID NO: 19) or (GGGGS)₃ linker (SEQ ID NO: 21) is linked to the IgM Fc region.

For gene cloning, after codon optimization of each of the material, codon-optimized nucleotide molecules were synthesized in Cosmogenetech Inc. (Seoul, Korea).

Insert DNA was prepared so that overlap PCR could be performed on the multiple cloning site (MCS) of pcDNA 3.1 (available from Thermo Scientific) vector using the optimized nucleotides and primers. After overlap PCR of the obtained insert DNAs and pcDNA 3.1 vectors, they were treated with DpnI restriction enzyme to remove the template, and each vector inserting each material was prepared.

After introducing the vector into Stella^{®} competent cell (available from Clontech), it was plated on an agar plate adding ampicillin antibiotics corresponding to the antibiotic resistance gene present in the vector, and then colonies were selected. The nucleotide sequence was confirmed through DNA sequencing using Applied biosystems 3730x1 DNA analyzer (available from Thermo Scientific).

### Example 1-2. Preparation of fusion protein comprising IgM Fc region, J chain, linker and biologically active molecule

In Example 1-2, a recombinant plasmid comprising a biologically active molecule was prepared and linked to the recombinant plasmid comprising the IgM Fc region, J chain and/or linker prepared in Example 1-1, and then a fusion protein containing these was expressed and purified.

As a biologically active molecule, a HLA-A, a IgG antibody fragment (IgG Fab) or an EPO analogue was used.

### Example 1-2-1. Fusion protein comprising HLA-A

A fusion protein comprising a HLA-A as a biologically active molecule, IgM Fc region, J chain and/or linker was prepared.

Specifically, in order to increase the stability of the HLA-A protein, cytomegalovirus (CMV)-derived peptide was additionally bound (loaded), and beta-2 microglobulin (β2m) was subsequently bound to the CMV-derived peptide. Accordingly, an HLA-A protein with improved stability (hereinafter referred to as 'CMVpHLA'; SEQ ID NO: 23) was constructed. In addition, CMVpHLAK^{b} (SEQ ID NO: 25) was prepared in which the α3 region of CMVpHLA was replaced with mouse K^{b}. In order to facilitate protein purification later, a FLAG tag was attached to the C-terminus, and the cloning method according to Example 1-1 was used.

In the constructed CMVpHLA recombinant plasmid, the CMVpHLA protein sequence except for the FLAG tag was linked to the IgM Fc region sequence. The final recombinant plasmid vector constructed in this way was introduced into Expi293 (available from Thermo Scientific) animal cells to establish a transient expression system for obtaining a fusion protein within a short time.

Specifically, one day before transformation, cells at a concentration of 3.0×10⁶ cells/mL were dispensed into a flask and cultured for 24 hours. On the day of transformation, Expi293^{®} expression medium was added to dilute cells to a concentration of 3.0×10⁶ cells/mL. Then, 1 µg/mL of each recombinant plasmid vector was mixed with Opti-MEM^{®} medium, and ExpiFectamine^{®} reagent, and Opti-MEM^{®} medium were mixed and left at room temperature for 5 minutes. After that, DNA and ExpiFectamine^{®} reagent were reacted for 15 minutes and added to the flask. They were treated with enhancer 1 and 2 after 16 to 22 hours. After confirming 75% or more of cell viability on the day 4 or 5, a fusion protein was recovered.

Then, the expressed fusion protein was purified using IgM affinity chromatography. Specifically, the culture solution obtained from the transient expression system was filtered through a PES filtration membrane having a pore size of 0.2 µm to remove impurities. The recovered filtrate was loaded onto the IgM affinity matrix POROS^{™} CaptureSelect^{™} IgM Affinity Matrix^{®} (available from Thermo Scientific) to purify the fusion protein. IgM affinity chromatography was performed under the following conditions:

### <Chromatography Conditions>

- Resin: POROS^{™} CaptureSelect^{™} IgM Affinity Matrix^{®}
- Flow rate: 480 cm/h
- Equilibration: Phosphate-buffered saline (PBS) buffer
- Loading: up to 6 g protein/L resin volume
- Regeneration and sterilization: 0.01 M NaOH solution
- Elution: 0.1 M glycine-HCl, pH 2.7 buffer
- Storage: 20% EtOH

Then, it was neutralized with 1.0 M Tris-HCl, pH 8.5 buffer in an amount of 1/10 of the volume of the eluate. The recovered fusion protein was concentrated using ultrafiltration, dialyzed against PBS, and then stored at -20 °C.

Meanwhile, purification and concentration of protein in the CMVpHLA control group not comprising the IgM Fc region were performed as follows. The culture solution obtained from the transient expression system was filtered through a PES filtration membrane having a pore size of 0.2 µm to remove impurities. The recovered filtrate was loaded on a DYKDDDDK (FLAG-tag) affinity matrix Pierce Anti-DYKDDDDK Affinity Resin^{®} (available from Thermo Scientific), and purified.

FLAG-tag affinity chromatography was performed under the following conditions:

### <Chromatography Conditions>

- Resin: Pierce Anti-DYKDDDDK Affinity Resin^{®}
- Flow rate: 300 cm/h
- Equilibration: Phosphate-buffered saline (PBS) buffer
- Loading: up to 3 g protein/L resin volume
- Regeneration and sterilization: 0.1 M glycine-HCl, pH 2.7 buffer
- Elution: 0.1 M glycine-HCl, pH 2.7 buffer
- Storage: 20% EtOH

After FLAG-tag affinity chromatography, it was neutralized with 1.0 M Tris-HCl, pH 8.5 buffer in an amount of 1/10 of the volume of the eluate. The recovered fusion protein was concentrated using ultrafiltration, dialyzed against PBS, and then stored at -20 °C.

The fusion protein prepared by the above method was represented by the structural formula: [(biologically active molecule)-(linker)-(IgM Fc region)]-(J chain). The number of biologically active molecules, linkers, IgM Fc regions and/or J chains comprised in one fusion protein is indicated as a subscript, but it is not indicated when the number is one.

**[Table 1]**

| **Fusion protein** | **Description** |
|---|---|
| [(CMVpHLA)₂-(IgD hinge region)₂-(IgM Fc region)]₅-(J chain) | The linkers are IgD hinge regions, and the IgM Fc regions form a pentamer. |
| [(CMVpHLA)₂-(IgA hinge region)₂-(IgM Fc region)]₅-(J chain) | The linkers are IgA hinge regions, and the IgM Fc regions form a pentamer. |
| [(CMVpHLA)₂-((GGGGS)₃)₂-(IgM Fc region)]₅-(J chain) | The linkers are (GGGGS)₃, and the IgM Fc regions form a pentamer. |
| [(CMVpHLA)₂-(linker)₀-(IgM Fc region)]s-(J chain) | Any linker is not comprised, and the IgM Fc regions form a pentamer. |
| [(CMVpHLA)₂-(IgD hinge region)₂-(IgM Fc region)]₆-(J chain)₀ | The linkers are IgD hinge regions, and the IgM Fc regions form a hexamer. |
| CMVpHLA | Any linker, IgM Fc region and J chain are not comprised. |
| CMVpHLAK^{b} | Any linker, IgM Fc region and J chain are not comprised, and α3 region is substituted with mouse K^{b}. |

In Table 1, for example, '[(CMVpHLA)₂-(IgD hinge region)₂-(IgM Fc region)]s-(J chain)' refers to a fusion protein in which two CMVpHLAs as biologically active molecules bind to an IgM Fc region monomer through two IgD hinge regions, respectively, and five IgM Fc region monomers form a pentamer with one J chain. FIGs. 1A and 11A may be referred as this pentamer structure of fusion protein.

In addition, '[(CMVpHLA)₂-(linker)₀-(IgM Fc region)]s-(J chain)' refers to a fusion protein in which two CMVpHLAs as biologically active molecules directly bind to an IgM Fc region monomer without a linker, and five IgM Fc region monomers form a pentamer with one J chain. FIG. 1B may be referred as this pentamer structure of fusion protein.

In addition, '[(CMVpHLA)₂-(IgD hinge region)₂-(IgM Fc region)]₆-(J chain)₀' refers to a fusion protein in which two CMVpHLAs as biologically active molecules bind to an IgM Fc region monomer through two IgD hinge regions, respectively, and six IgM Fc region monomers form a hexamer without J chain. FIG. 1C may be referred as this hexamer structure of fusion protein.

### Example 1-2-2. Fusion protein comprising IgG antibody fragment

A fusion protein comprising an IgG antibody fragment as a biologically active molecule, IgM Fc region, J chain and/or linker was prepared.

Specifically, a portion of the antibody that binds to the HLA-G protein (IgG Fab; hereinafter referred to as 'A2014') was used. The A2014 was constructed from the light chain (VL domain and CL domain; SEQ ID NO: 27) and the part of heavy chain (VH domain and CH1 domain; SEQ ID NO: 31) among the IgG Fab moieties using the cloning method according to Example 1-1.

In addition, a fusion protein comprising an IgG Fc region instead of an IgM Fc region was prepared as a control. As the IgG Fc region, the CH2 and CH3 domains of IgG were used, specifically the light chain (VL domain and CL domain; SEQ ID NO: 27) and the heavy chain (VH domain, CH1 domain, CH2 domain and CH3 domain; SEQ ID NO: 29). It was prepared using the cloning method according to Example 2-1.

The constructed A2014 recombinant plasmid sequence was linked to the IgM Fc region or IgG Fc region sequence. The final recombinant plasmid vector constructed in this way was introduced into the cells according to the method performed in Example 1-2-1, and the fusion protein was expressed.

Then, a culture solution was purified and concentrated, dialyzed against PBS, and stored at -20 °C according to the same method as in Example 1-2-1.

Meanwhile, purification and concentration of the fusion protein comprising the IgG Fc region were performed as follows. The culture solution obtained from the transient expression system was loaded on an IgG affinity matrix MabSelect SuRe^{®} (Cytiva) and purified according to the method of Example 1-2-1.

IgG affinity chromatography was performed under the following conditions:

### <Chromatography Conditions>

- Resin: MabSelect SuRe^{®}
- Flow rate: 300 cm/h
- Equilibration: Phosphate-buffered saline (PBS) buffer
- Loading: up to 35 g protein/L resin volume
- Regeneration and sterilization: 0.01 M NaOH solution
- Elution: 0.1 M glycine-HCl, pH 2.7 buffer
- Storage: 20% EtOH

After the IgG affinity chromatography, it was neutralized with 1.0 M Tris-HCl, pH 8.5 buffer in an amount of 1/10 of the volume of the eluate. The recovered fusion protein was concentrated using ultrafiltration, dialyzed against PBS, and then stored at -20 °C.

**[Table 2]**

| **Fusion protein** | **Description** |
|---|---|
| [(A2014)₂-(IgD hinge region)₂-(IgM Fc region)]s-(J chain) | The linkers are IgD hinge regions, and the IgM Fc regions form a pentamer. |
| [(A2014)₂-(IgA hinge region)₂-(IgM Fc region)]s-(J chain) | The linkers are IgA hinge regions, and the IgM Fc regions form a pentamer. |
| [(A2014)₂-((GGGGS)₃)₂-(IgM Fc region)]s-(J chain) | The linkers are (GGGGS)₃, and the IgM Fc regions form a pentamer. |
| [(A2014)₂-(linker)₀-(IgM Fc region)]₅-(J chain) | Any linker is not comprised, and the IgM Fc regions form a pentamer. |
| [(A2014)₂-(IgD hinge region)₂-(IgM Fc region)]e-(J chain)₀ | The linkers are IgD hinge regions, and the IgM Fc regions form a hexamer. |
| (A2014)₂-(IgG Fc region) | Any linker, IgM Fc region and J chain are not comprised, but the IgG Fc region is comprised. |

The fusion proteins prepared by the above method are shown in Table 2. For example, '[(A2014)₂-(IgD hinge region)₂-(IgM Fc region)]s-(J chain)' is a fusion protein in which two A2014s as biologically active molecules bind to an IgM Fc region monomer through two IgD hinge regions, respectively, and five IgM Fc region monomers form a pentamer with one J chain. FIGs. 1A and 11A may be referred as this pentamer structure of fusion protein.

In addition, '[(A2014)₂-(linker)₀-(IgM Fc region)]s-(J chain)' is a fusion protein in which two A2014s as biologically active molecules directly bind to an IgM Fc region monomer without a linker, and five IgM Fc region monomers form a pentamer with one J chain. FIG. 1B may be referred as this pentamer structure of fusion protein.

In addition, '[(A2014)₂-(IgD hinge region)₂-(IgM Fc region)]₆-(J chain)₀' is a fusion protein in which two A2014s as biologically active molecules bind to an IgM Fc region monomer through two IgD hinge regions, respectively, and six IgM Fc region monomers form a hexamer without J chain. FIG. 1C may be referred as this hexamer structure of fusion protein.

### Example 1-2-3. Fusion protein comprising EPO analogue

A fusion protein comprising EPO analogue as a biologically active molecule, IgM Fc region, J chain and/or linker was prepared.

Specifically, a novel erythropoiesis stimulating protein (NESP) protein was used as an EPO analogue. The NESP (SEQ ID NO: 33) was linked to the IgM Fc region sequence, and a plasmid vector was constructed using the cloning method according to Example 1. The constructed recombinant plasmid vector was introduced into cells according to the method performed in Example 1-2-1, fusion protein was expressed, purified, and concentrated, and then dialyzed against PBS and stored at -20 °C.

**[Table 3]**

| **Fusion protein** | **Description** |
|---|---|
| [(NESP)₂-(IgD hinge region)₂-(IgM Fc region)]₅-(J chain) | The linkers are IgD hinge regions, and the IgM Fc regions form a pentamer. |
| [(NESP)₂-(linker)₀-(IgM Fc region)]₅-(J chain) | Any linker is not comprised, and the IgM Fc regions form a pentamer. |
| NESP | Any linker, IgM Fc region and J chain are not comprised. |

The fusion proteins prepared by the above method are shown in Table 3. For example, '[(NESP)₂-(IgD hinge region)₂-(IgM Fc region)]s-(J chain)' is a fusion protein in which two NESPs as biologically active molecules bind to an IgM Fc region monomer through two IgD hinge regions, respectively, and five IgM Fc region monomers form a pentamer with one J chain. FIGs. 1A and 11A may be referred as this pentamer structure of fusion protein.

In addition, '[(NESP)₂-(linker)₀-(IgM Fc region)]s-(J chain)' is a fusion protein in which two NESPs as biologically active molecules directly bind to an IgM Fc region monomer without a linker, and five IgM Fc region monomers form a pentamer with one J chain. FIG. 1B may be referred as this pentamer structure of fusion protein.

### Example 1-3. Structure of the fusion protein

It was confirmed whether the fusion protein prepared in Example 1-2 has the desired structure. A fusion protein comprising a biologically active molecule, a linker, an IgM Fc region monomer, and a J chain, in which the IgM Fc region forms a pentamer, is shown in FIGs. 1A and 11A. In addition, a fusion protein comprising a biologically active molecule, an IgM Fc region monomer, and a J chain, but not a linker, in which the IgM Fc region forms a pentamer, is shown in FIG. 1B. In addition, a fusion protein comprising a biologically active molecule, a linker, and an IgM Fc region monomer, but not a J chain, in which the IgM Fc region forms a hexamer, is shown in FIG. 1C. In addition, a fusion protein comprising a biologically active molecule and an IgM Fc region monomer but not a linker and a J chain, in which the IgM Fc region forms a hexamer, is shown in FIG. 1D.

Specifically, the structure of the fusion protein was confirmed using Reducing SDS-PAGE. After mixing 5x reducing-PAGE sample buffer with 2 µg of each fusion protein, it was heated at 100 °C for 5 minutes, and then left at room temperature for 20 minutes. After inserting polyamide gel (12-20%, Invitrogen) into the gel kit, loading the sample, and electrophoresing it at 200V for 30 minutes, the gel was separated from the kit and a staining solution (Coomassie Brilliant Blue staining solution) was added. Then, it was left for 1 hour. The left gel was transferred to a buffer (destaining buffer; 70% tertiary distilled water, 20% methanol, 10% acetic acid), and the result was confirmed after overnight.

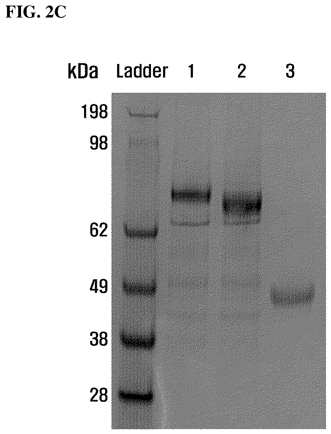

As a result, as shown in FIG. 2A, FIG. 2B and FIG. 2C, each of the fusion protein comprising IgM Fc region, biologically active molecule, J chain and/or linker was confirmed to contain all sizes corresponding to components that constitute it.

The results show that the fusion proteins prepared in Example 1-2 have the desired structure and are normally expressed.

### Example 1-4. Target binding capacity of the fusion protein

In Example 1-4, the target binding capacity of the fusion protein prepared in Example 1-2 was analyzed. The target was set differently depending on the biologically active molecule (CMVpHLA, IgG antibody fragment or EPO analogue) comprised in each fusion protein.

### Example 1-4-1. Fusion protein comprising CMVpHLA

A binding capacity to an anti-CMVpHLA antibody, which is a target of the fusion protein comprising CMVpHLA as a biologically active molecule, was confirmed.

Specifically, it was analyzed through ELISA assay and Surface Plasmon Resonance (SPR).

The ELISA assay was performed as follows. A 96-well plate was coated with anti-CMVpHLA antibody (available from Ajou University) at 10 ng/well at 4°C for 16 hours. Then, the plate was washed with 0.05% PBS-Tween and blocked with 3% skim milk. After blocking, a dilution containing each fusion protein or control protein was added to the well and incubated at 37°C for 2 hours. Then, the plate was washed, and HRP-conjugated anti-IgM (F5µ) antibody (available from MERCK) or HRP-conjugated anti-FLAG antibody (available from Abcam) was diluted 1:5000 and incubated at 37°C for 1 hour. Thereafter, the plate was washed nine times with 0.05% PBS-Tween, and then developed with TMB solution (available from Sigma). After that, the reaction was terminated with 1N sulfuric acid (available from Daejung Chemicals & Metals) and analyzed by VARIOSKAN LUX (available from Thermo Scientific).

SPR analysis was performed as follows. Biacore T200 available from Cytiva was used as an SPR analyzer. To label anti-CMVpHLA mouse Fc antibody (available from Ajou University) on CM5 chip (available from Cytiva), recombinant protein G (available from Abcam) was attached using EDC/NHS reaction and blocked with 1M ethanolamine. Then, CMVpHLA mouse Fc antibody was flowed and coated on the chip in a directional manner. After that, a dilution containing each fusion protein or control protein was flowed, and the dissociation reaction was performed with 10 mM glycine-HCl, pH 1.5.

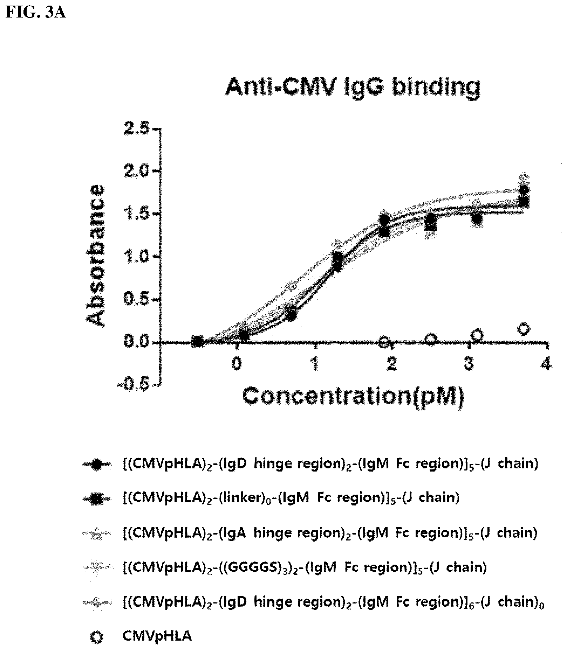

As a result, as shown in FIG. 3A, the CMVpHLA control protein not comprising IgM Fc region had a very low binding activity to the target (anti-CMVpHLA antibody). On the contrary, the fusion protein comprising IgM Fc region had a very high binding activity to the target (anti-CMVpHLA antibody), and the EC₅₀ value was improved several tens of times compared to the CMVpHLA control protein.

**[Table 4]**

| **Fusion protein** | **ka (1/Ms)** | **kd (1/s)** | **KD (M)** | **Rmax** | **Avidity score** |
|---|---|---|---|---|---|
| [(CMVpHLA)₂-(IgD hinge region)₂-(IgM Fc region)]₅-(J chain) | 5.56×10⁶ | 3.78×10⁻⁵ | 6.81×10⁻¹² | 33.72 | 8.92×10⁵ |
| [(CMVpHLA)₂-(linker)₀-(IgM Fc region)]₅-(J chain) | 5.44×10⁶ | 5.56×10⁻⁵ | 1.02×10⁻¹¹ | 33.6 | 6.05×10⁵ |
| [(CMVpHLA)₂-((GGGGS)₃)₂-(IgM Fc region)]₅-(J chain) | 4.74×10⁶ | 2.94×10⁻⁵ | 6.22×10⁻¹² | 32.06 | 1.09×10⁶ |
| [(CMVpHLA)₂-(IgA hinge region)₂-(IgM Fc region)]₅-(J chain) | 5.43×10⁶ | 3.18×10⁻⁵ | 5.86×10⁻¹² | 30.2 | 9.50×10⁵ |
| [(CMVpHLA)₂-(IgD hinge region)₂-(IgM Fc region)]₆-(J chain)₀ | 8.44×10⁶ | 1.97×10⁻⁴ | 2.33×10⁻¹¹ | 29.88 | 1.52×10⁵ |
| CMVpHLA | 2.52×10⁶ | 0.004645 | 1.84×10⁻⁹ | 10.33 | 2.22×10³ |

| | | | | | |
|---|---|---|---|---|---|
| * In Table 4, ka refers to a rate at which two substances are associated (association rate constant), kd refers to a rate at which two substances are dissociated (dissociation rate constant), KD refers to a binding affinity, Rmax refers to a maximum RU value at which the angle in SPR varies when two substances are bound, and Avidity score refers to a Rmax/kd value indicating a degree of avidity in SPR. | | | | | |

In addition, as shown in Table 4, the CMVpHLA control protein not comprising IgM Fc region had a very low KD value and avidity score (Rmax/Kd) for the target (CMVpHLA mouse Fc antibody). On the contrary, the fusion protein comprising IgM Fc region had significantly higher KD value and avidity score (Rmax/Kd) for the target (CMVpHLA mouse Fc antibody), and the binding capacity was improved by 100 to 1000 times compared to the CMVpHLA control protein. The results show that the IgM Fc region can improve the binding capacity of the biologically active molecule to the target, and thus the fusion protein comprising IgM Fc region and biologically active molecule according to the present invention can exhibit a high level of desired biological activity in the cell.

### Example 1-4-2. Fusion protein comprising IgG antibody fragment

A binding capacity to a HLA-G protein antigen, which is a target of the fusion protein comprising IgG antibody fragment (IgG Fab; A2014) as a biologically active molecule, was confirmed.

Specifically, it was analyzed through ELISA assay and SPR according to Example 1-4-1.

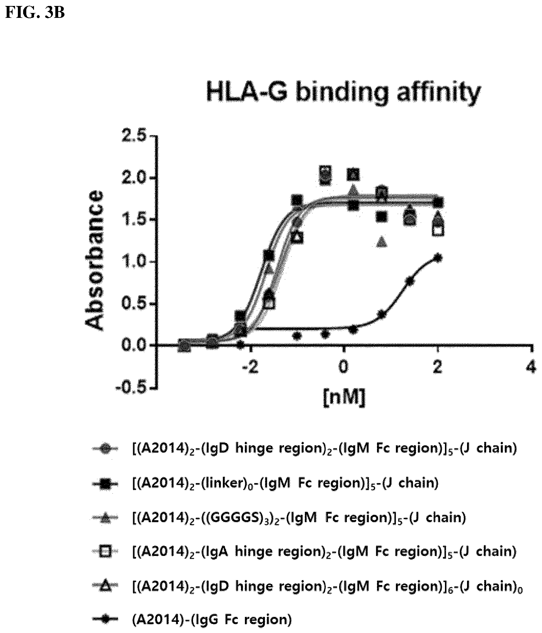

As a result, as shown in FIG. 3B, the (A2014)-(IgGFc) control protein comprising IgG Fc region had a very low binding activity to the HLA-G antigen. On the contrary, the fusion protein comprising IgM Fc region had very high binding activity to the HLA-G antigen, and the EC₅₀ value was improved about 1000 times or more compared to the (A2014)-(IgGFc) control protein.

**[Table 5]**

| **Fusion protein** | **ka (1/Ms)** | **kd (1/s)** | **KD (M)** | **Rmax** | **Avidity score** |
|---|---|---|---|---|---|
| [(A2014)₂-(IgD hinge region)₂-(IgM Fc region)]₅-(J chain) | 9.58×10⁶ | 4.25×10⁻⁵ | 4.44×10⁻¹² | 93.38 | 2.20×10⁶ |
| [(A2014)₂-(linker)₀-(IgM Fc region)]₅-(J chain) | 2.63×10⁷ | 1.12×10⁻⁴ | 4.25×10⁻¹² | 59.87 | 5.36×10⁵ |
| [(A2014)₂-((GGGGS)₃)₂-(IgM Fc region)]₅-(J chain) | 1.96×10⁷ | 9.66×10⁻⁵ | 4.94×10⁻¹² | 56.68 | 5.87×10⁵ |
| [(A2014)₂-(IgA hinge region)₂-(IgM Fc region)]₅-(J chain) | 1.39×10⁷ | 5.05×10⁻⁵ | 3.63×10⁻¹² | 73.38 | 1.45×10⁶ |
| [(A2014)₂-(IgD hinge region)₂-(IgM Fc region)]₆-(J chain)₀ | 1.48×10⁷ | 3.51×10⁻⁵ | 2.32×10⁻¹² | 86.71 | 2.47×10⁶ |
| (A2014)-(IgG Fc) | 1.18×10⁷ | 3.05×10⁻⁴ | 2.59×10⁻¹¹ | 17.44 | 5.71×10⁴ |

| | | | | | |
|---|---|---|---|---|---|
| * In Table 5, ka refers to a rate at which two substances are associated (association rate constant), kd refers to a rate at which two substances are dissociated (dissociation rate constant), KD refers to a binding affinity, Rmax refers to a maximum RU value at which the angle in SPR varies when two substances are bound, and Avidity score refers to a Rmax/kd value indicating a degree of avidity in SPR. | | | | | |

In addition, as shown in Table 5, the (A2014)-(IgG Fc) control protein comprising IgG Fc region had a very low KD value and avidity score (Rmax/Kd) for the HLA-G antigen. On the contrary, the fusion protein comprising IgM Fc region had a very high KD value and avidity score (Rmax/Kd) for the HLA-G antigen, and the binding capacity was improved by 10 to 500 times compared to the (A2014)-(IgGFc) control protein. The results show that the IgM Fc region can improve the binding capacity of the biologically active molecule to the target, and thus the fusion protein comprising IgM Fc region and biologically active molecule according to the present invention can exhibit a high level of desired biological activity in the cell.

### Example 1-4-3. Fusion protein comprising EPO analogue

A binding capacity to an EPO receptor, which is a target of the fusion protein comprising EPO analogue (NESP) as a biologically active molecule, was confirmed.

Specifically, it was analyzed through ELISA assay and SPR according to Example 1-4-1.

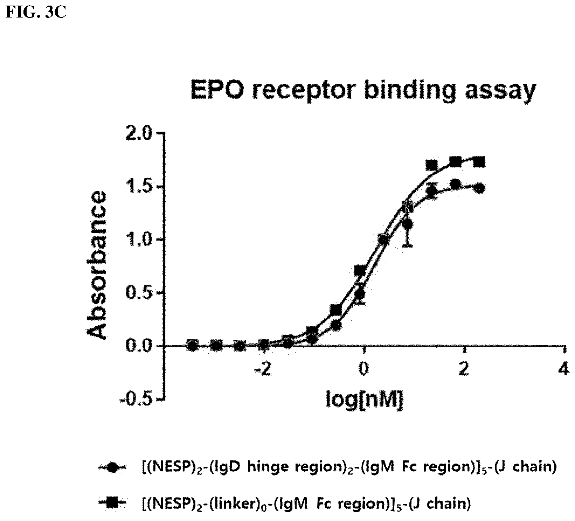

As a result, as shown in FIG. 3C, the fusion protein comprising IgM Fc region and NESP had a very high binding activity to the EPO receptor.

**[Table 6]**

| **Fusion protein** | **ka (1/Ms)** | **kd (1/s)** | **KD (M)** | **Rmax** | **Avidity score** |
|---|---|---|---|---|---|
| [(NESP)₂-(IgD hinge region)₂-(IgM Fc region)]₅-(J chain) | 1.383×10⁶ | 1.240×10⁻⁵ | 8.968×10⁻¹² | 402.0 | 3.24×10⁷ |
| [(NESP)₂-(linker)₀-(IgM Fc region)]₅-(J chain) | 1.612×10⁶ | 4.051×10⁻⁵ | 2.513×10⁻¹¹ | 354.8 | 8.76×10⁶ |
| NESP | 1.241×10⁶ | 1.456×10⁻³ | 1.173×10⁻⁹ | 111.2 | 7.64×10⁴ |

| | | | | | |
|---|---|---|---|---|---|
| * In Table 6, ka refers to a rate at which two substances are associated (association rate constant), kd refers to a rate at which two substances are dissociated (dissociation rate constant), KD refers to a binding affinity, Rmax refers to a maximum RU value at which the angle in SPR varies when two substances are bound, and Avidity score refers to a Rmax/kd value indicating a degree of avidity in SPR. | | | | | |

In addition, as shown in Table 6, the NESP control protein not comprising IgM Fc region had a very low KD value and avidity score (Rmax/Kd) for the EPO receptor. On the contrary, the fusion protein comprising IgM Fc region and NESP had a very high KD value and avidity score (Rmax/Kd) for the EPO receptor, and the binding ability was improved by 100 to 400 times compared to the NESP control protein. The results show that the IgM Fc region can improve the binding capacity of the biologically active molecule to the target, and thus the fusion protein comprising IgM Fc region and biologically active molecule according to the present invention can exhibit a high level of desired biological activity in the cell.

In particular, the results of Examples described above show that the effect of fusion protein when the biologically active molecule binds to the IgM Fc region is obtained regardless of Mode of Action (MoA) or the type of biologically active molecule.

### Example 1-5. Biological activity of fusion protein

### Example 1-5-1. Activation, proliferation inducing ability and cytotoxicity of CD8⁺ T cells

In order to confirm a biological activity of the fusion protein prepared in Example 1-2, an activation and a proliferation inducing ability of CD8⁺ T cells were analyzed.

The activation for CD8⁺ T cells was confirmed by IFN-γ ELISPOT assay. PBMC were purchased from Immunospot, CD8⁺ T cells were obtained using a CD8⁺ T cell isolation kit (available from Miltenyi Biotec), and ELISPOT analysis was performed using an ELISPOT kit (available from Immunospot). Then, after washing the IFN-γ antibody-coated plate with PBS, RPMI-1640 (10% FBS) was added to stabilize the antibody. After removing RPMI-1640, a dilution containing each fusion protein or control protein was added to the plate, respectively. Then, PBMCs (6×10⁵ cells/well) and CD8⁺ T cells (2×10⁵ cells/well) were put into each well containing the fusion protein and reacted in a 37°C 5% CO₂ incubator for 48 hours. The reacted plate was washed with PBS to remove cells and materials, and then reacted with a biotinylated IFN-γ antibody at room temperature for 2 hours. After washing with PBS, Strep-AP was added and reacted at room temperature for 1 hour, washed with PBS, and reacted with the substrate for 10 minutes to induce color development. After that, it was analyzed with an ELISPOT reader (AID) and statistical processing was performed using the GraphPad Prism Version 5.0 (GraphPad software) analysis program.

The ability to induce the proliferation of CD8⁺ T cells was confirmed by FACS. PBMCs, purchased from Immunospot, 1×10⁶ cells/mL were suspended in RPMI-1640 (10% FBS), and seeded in 12-well at a density of 1×10⁶ cells/well. Then, each fusion protein or control protein was treated at a concentration of 2 nM per well, and 20 U/mL of IL-2 (Peprotech) was added and cultured. After 3 to 4 days, IL-2 20 U/mL, IL-7 (Peprotech) 25 ng/mL, and IL-15 (Peprotech) 25 ng/mL were added, and on the 7th to 8th days of culture, the three types of cytokines were mixed, and a culture medium was added as much as the initial volume. Each cell was harvested on days 10 ~ 11 or 19 ~ 20 and washed with FACS buffer (5% FBS in PBS). Thereafter, the cell suspension was treated with an isotype antibody (Biolegend) to perform Fc blocking, followed by washing with FACS buffer. Then, it was washed after treatment with PerCP cy5.5 anti-human CD8 antibody (BD bioscience) and washed after treatment with HLA-A*02:01 CMV pp65 tetramer (MBL). After completion of the process, the proliferation of CMV-specific CD8⁺ T cells was checked using a flow cytometer (BD bioscience, FACSLyric^{™}).

In addition, the analysis for the cytotoxicity of CD8⁺ T cells was confirmed by FACS. PBMCs, purchased from Immunospot, 6×10⁵ cells/mL were suspended in RPMI-1640 (10% FBS), and seeded into 6-well at a density of 1.8×10⁶ cells/well. After seeding, each fusion protein or control protein was treated at a concentration of 100 nM per well, and 20 U/mL of IL-2 (Peprotech) and 2 µg/mL of anti-human CD28 antibody (Ebioscience) were added and cultured. After 3 to 4 days, IL-2 20 U/mL, IL-7 (Peprotech) 25 ng/mL, IL-15 (Peprotech) 25 ng/mL, and anti-human CD28 antibody 2 µg/mL were added and cultured. On the 7th to 8th days, the three types of cytokines and one type of antibody were mixed, and each fusion protein or control protein was added at the same time as adding the same amount of culture medium as the initial volume. Then, every 3 to 4 days, the three types of cytokines and one type of antibody were mixed and added, and on days 19 to 20, each cell was washed with FACS buffer, and then CD8⁺ T cells were obtained using a CD8⁺ T cell isolation kit (Miltenyi Biotec). After that, the target cells, T2 cells (ATCC), were reacted with 50 µg/mL of CMVpp65 peptide and 3 µg/mL of B2M (Sino) at 37 °C in a 5% CO₂ incubator for 1 hour. After washing with FACS buffer, 1×10⁶ cells/4 µM DiO (Invitrogen) was treated, and the reaction was performed in a 5% CO₂ incubator at 37 °C, followed by washing. Thereafter, the DiO-labeled T2 cells and the CD8⁺ T cells were mixed in each ratio, and each fusion protein or control protein was added thereto, followed by reaction for 4 hours. And then, 7AAD (Invitrogen) was treated. After 10 minutes of reaction, the degree of apoptosis of T2 cells was analyzed by the flow cytometry.

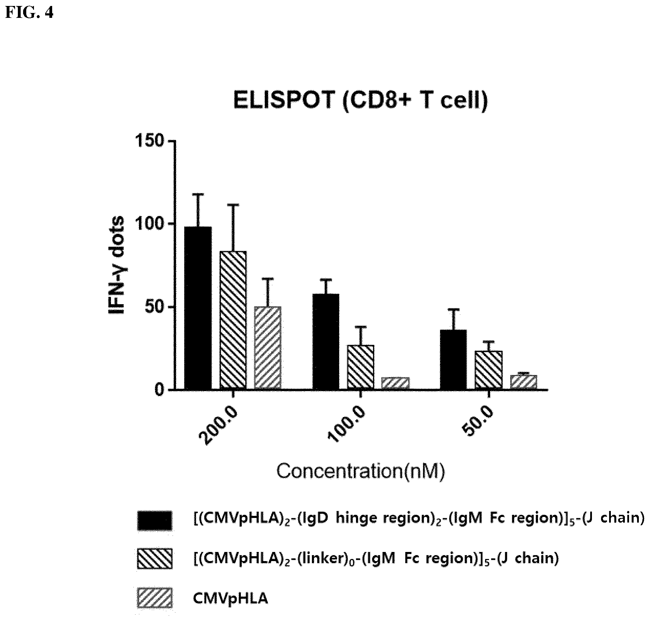

As a result, as shown in FIG. 4, the fusion protein comprising IgM Fc region and CMVpHLA increased the production of IFN-γ by about 2 times or more compared to the CMVpHLA control protein not comprising IgM Fc region.

In addition, compared to the fusion protein comprising IgM Fc region but not a linker, the fusion protein comprising both IgM Fc region and linker exhibited a higher level of the production of IFN-γ. The results suggest that the target binding ability of the fusion protein increases due to IgM Fc, and that this shows a better target binding ability when the linker is connected.

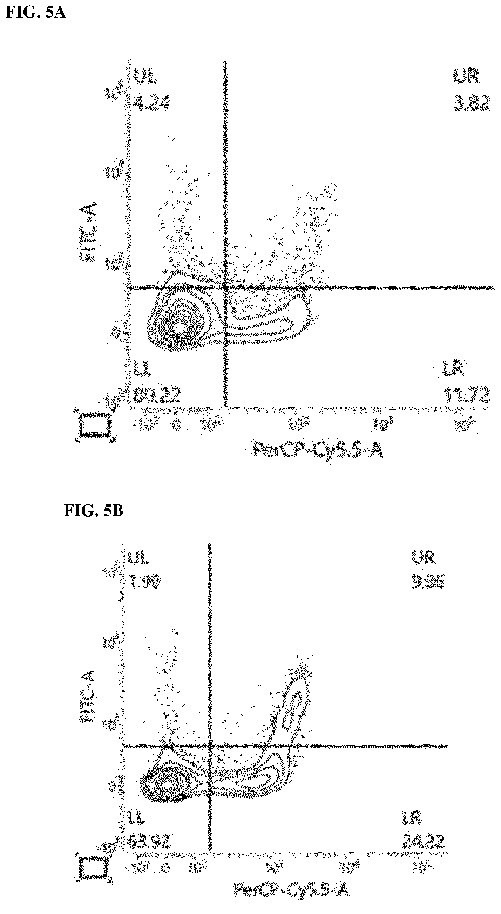

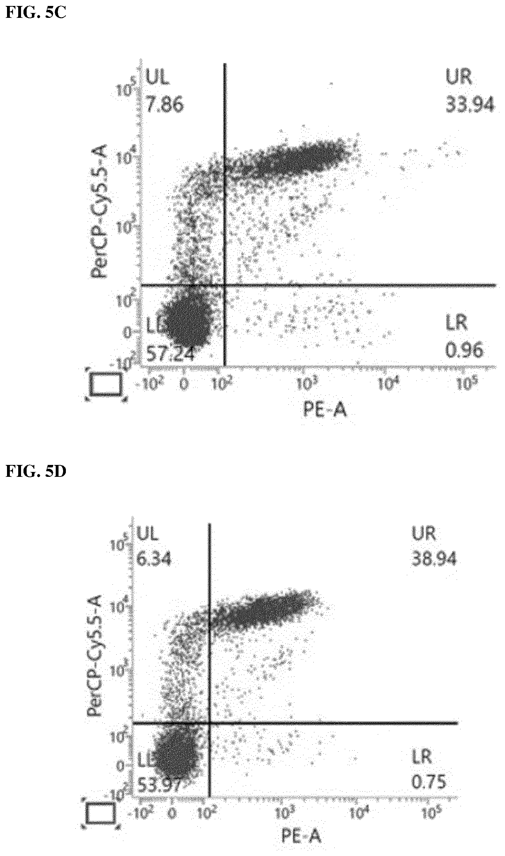

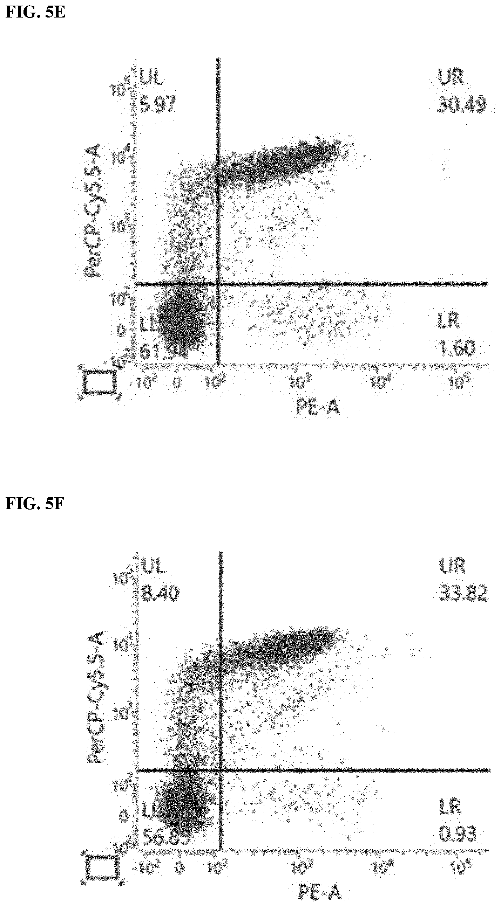

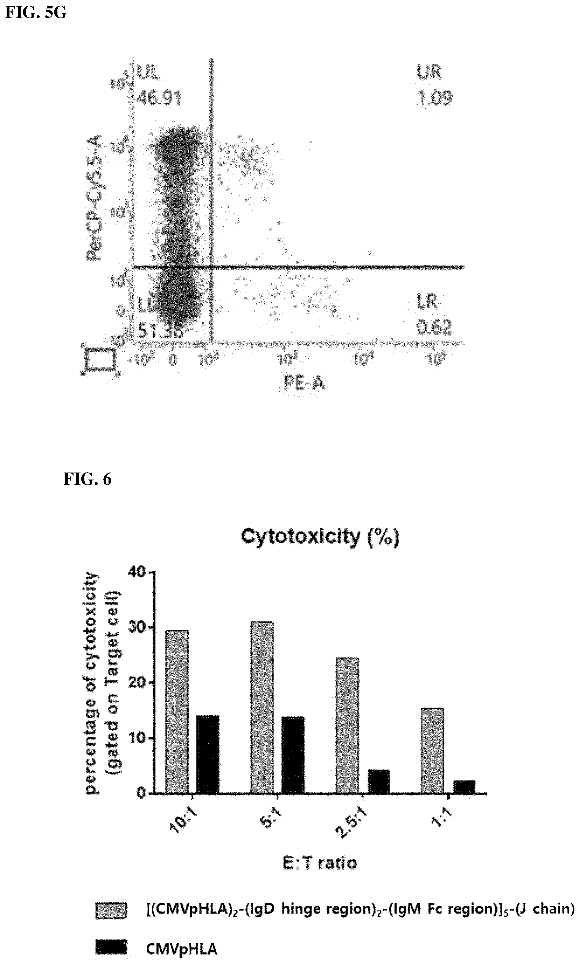

In addition, as shown in FIG. 5A, compared to the fusion protein comprising IgM Fc region but not a linker, the fusion protein comprising both IgM Fc region and linker was more than three times effective in inducing proliferation of CD8⁺ T cells.

In addition, as shown in FIG. 5B, the fusion protein comprising IgM Fc region, linker and CMVpHLA was more than 30 times effective in inducing proliferation of CD8⁺ T cells compared to the CMVpHLA control protein not comprising IgM Fc region.

Furthermore, as shown in FIG. 6, the fusion protein comprising IgM Fc region and CMVpHLA had higher toxicity to cancer cells compared to the CMVpHLA control protein not comprising IgM Fc region.

The results show that the fusion protein comprising IgM Fc region can induce the activation and proliferation of CD8⁺ T cells and death of cancer cells at a higher level than the protein not comprising IgM Fc region. In particular, it was found that when the IgM Fc region and the biologically active molecule are linked by a linker, it can show higher biological activity.

### Example 1-5-2. Binding activity for HLA-G expressing cell

In order to confirm the biological activity of the fusion protein prepared in Example 1-2, a binding capacity to JEG-3 cells expressing HLA-G was analyzed.

Specifically, a dilution containing each fusion protein or control protein was added to JEG-3 cells (ATCC, 3.5×10⁵ cells/100 µl) that naturally express HLA-G and reacted at 4 °C for 1 hour. After washing with FACS buffer (5% FBS in PBS), anti-IgM-PE (ebioscience) or anti-IgG Fc-FITC (Biolegend) was treated and reacted at 4°C for 30 minutes. Finally, after washing with FACS buffer, the degree of the fusion protein and control binding to the JEG-3 cells were confirmed using a flow cytometer (BD bioscience, FACSLyric^{™}).

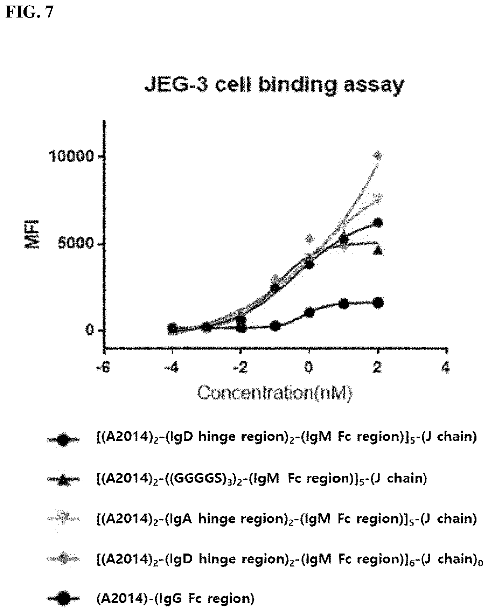

As a result, as shown in FIG. 7, the (A2014)-(IgG Fc) control protein comprising IgG Fc region had a very low binding activity to HLA-G expressing cells. On the contrary, the fusion protein comprising the IgM Fc region had a very high binding activity to the cells, and the maximum MFI (median fluorescence intensity) value was improved about 2.5 times or more compared to the (A2014)-(IgG Fc) control protein.

Through the above results, it was confirmed that the fusion protein comprising IgM Fc region had a higher activity binding to the receptor in cells than the protein not comprising IgM Fc region. Therefore, the fusion protein comprising IgM Fc region and biologically active molecule according to the present invention can show a high level of desired biological activity in cells.

### Example 1-5-3. Activity blocking the binding of ILT-2 protein

In order to confirm the biological activity of the fusion protein prepared in Example 1-2, the activity blocking the binding of an HLA-G protein and a ILT-2 (LILRB1) protein was analyzed. The ILT-2 protein is a receptor protein expressed in immune cells, and when binding to the HLA-G protein, the activities of cytotoxic T cells, NK cells and B cells are suppressed, so that a normal immune response is not occurred.

First, the activity blocking the binding between a recombinant HLA-G protein and a recombinant ILT-2 protein was analyzed by ELISA assay. ILT-2 antigen (R&D systems) was put in a 96-well plate at 150 ng/well, and then coated at 4°C for 16 hours. A plate was washed with 0.05% PBS-Tween and blocked with 3% skim milk. After blocking, a dilution containing each fusion protein or control protein was added to the wells, and HLA-G-6x his tag (IMBiologics) was treated at a predetermined concentration in each well and incubated at 37°C for 2 hours. Then, the plate was washed, and HRP-conjugated anti-6x his antibody (available from abcam) was diluted 1:10,000 and incubated at 37 °C for 1 hour. Thereafter, the plate was washed nine times with 0.05% PBS-Tween, developed with TMB solution (available from Sigma), and then the reaction was terminated with 1N sulfuric acid (available from Daejung Chemicals & Metals) and analyzed by VARIOSKAN LUX (available from Thermo Scientific).

The activity blocking the binding between the ILT-2 protein and the HLA-G protein naturally expressed in cells was analyzed using JEG-3 cells. JEG-3 cells (3×10⁵ cells/tube) purchased from ATCC were suspended with MEM containing 10% FBS. Then, a dilution containing each fusion protein or control protein was added to the tube and reacted at 4 °C for 30 minutes. After washing with FACS buffer (5% FBS in PBS), 1.5 µg of ILT-2 Fc chimera (available from R&D systems) was treated and reacted at 4 °C for 30 minutes. After washing with FACS buffer, it was treated with PE-anti-human IgG Fc (available from Biolegnd) and reacted at 4 °C for 20 minutes. Finally, after washing with FACS buffer, the degree of blocking the binding of the ILT-2 protein and the HLA-G protein expressed in cells was confirmed using a flow cytometer (BD bioscience, FACSLyric^{™}).

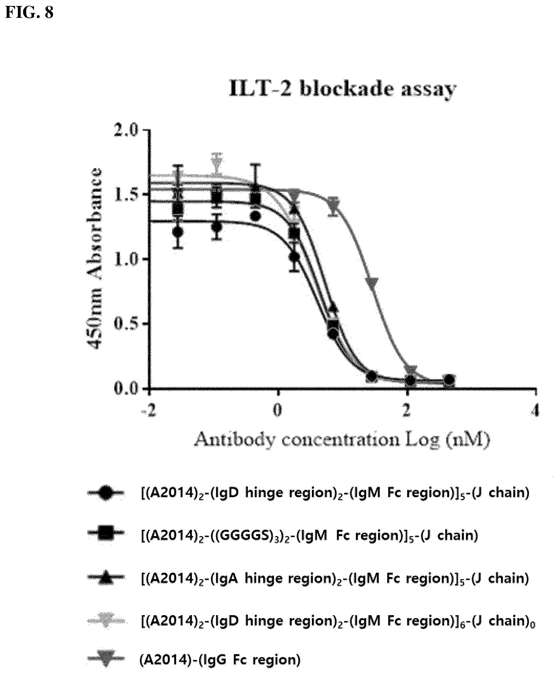

As a result, as shown in FIG. 8, the (A2014)-(IgG Fc) control protein comprising IgG Fc region had a very low activity in blocking the binding of the recombinant HLA-G protein and the ILT-2 protein. On the contrary, the fusion protein comprising IgM Fc region had a very high activity in blocking binding of the proteins, and the IC₅₀ value was improved about 5 to 7 times or more compared to the (A2014)-(IgG Fc) control protein.

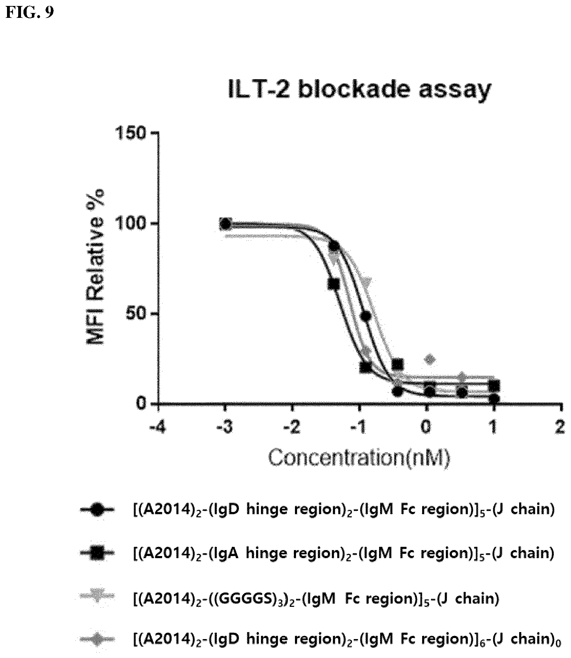

In addition, as shown in FIG. 9, the fusion protein comprising IgM Fc region has a very high activity in blocking the binding to the HLA-G protein expressed in the cells. This suggests that it can effectively suppress the binding of HLA-G expressed on the surface of cancer cells in the body to ILT-2 which is an inhibitory receptor of immune cells, thereby increasing the death of cancer cells.

From the results, it was confirmed that the fusion protein comprising IgM Fc region has a higher activity blocking the binding of the antigen and the receptor protein expressed in the cell, compared to the protein not comprising IgM Fc region. Therefore, the fusion protein comprising IgM Fc region and biologically active molecule according to the present invention can show the desired biological activity at a high level in the cell.

### Example 1-5-4. Binding activity with EPO receptor in cells

In order to confirm a biological activity of the fusion protein prepared in Example 1-2, an activity binding to cells expressing the EPO receptor was analyzed.

Specifically, the binding activity was confirmed using the EPOR reporter assay purchased from Indigobioscience. Cells were seeded in a 96-well cell culture plate (available from Coming) at a density of 150 µL/well, and then cultured in a 5% CO₂ incubator at 37°C for 4 to 6 hours. Then, a dilution containing each fusion protein or control protein was added to the wells, and incubated for one day in a 5% CO₂ incubator at 37°C. According to the manufacturer's protocol, 100 µL of the reaction solution was added, and after 5 minutes of color development at room temperature, analysis was performed with VARIOSKAN LUX (available from Thermo Scientific).

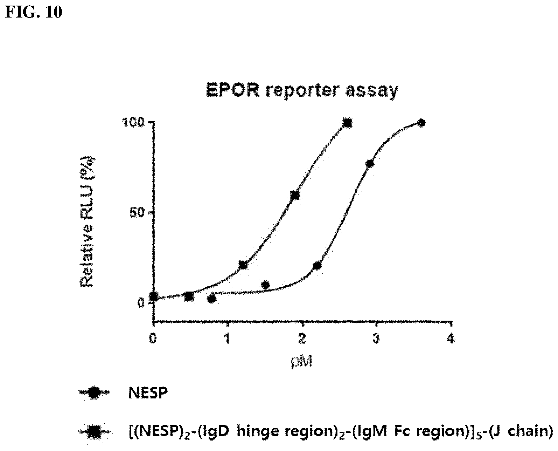

As a result, as shown in FIG. 10, the fusion protein comprising IgM Fc region and linker induced the proliferation of EPOR-overexpressed cells to a higher level compared to the NESP control protein not comprising IgM Fc region. That is, it was confirmed that the fusion protein comprising IgM Fc region shows an excellent binding activity with the EPO receptor in cells, and the signaling pathway by EPO receptor stimulation is activated, resulting in the improvement of cell proliferation ability.

From the above results, it was confirmed that the fusion protein comprising IgM Fc region has a higher ability binding to an intracellular receptor and a higher biological activity inducing ability, compared to the protein not comprising IgM Fc region. Therefore, the fusion protein comprising IgM Fc region and biologically active molecule according to the present invention can show a high level of desired biological activity in cells.

In particular, the results of Examples above suggest that the effect inducing the biological activity of the fusion protein by the IgM Fc region is obtained regardless of the MoA or type of biologically active molecule, when the biologically active molecule binds to the IgM Fc region.

### Example 2. Preparation of fusion protein platform comprising IgG Fc region and IgM Fc region

In this Example 2, a fusion protein platform comprising a biologically active molecule, linker, IgG Fc region, IgM Fc region and/or J chain was prepared. In addition, a fusion protein platform was prepared using mutations in which amino acids of the Fc region of wild-type IgG, the Fc region of wild-type IgM, and/or the wild-type J chain were deleted or substituted.

### Example 2-1. Preparation of wild-type fusion protein platform

In this Example 2-1, recombinant plasmids of linker, IgG Fc region, IgM Fc region and J chain were prepared. The linker was prepared using the IgD hinge region (part of the IgD CH1 domain, hinge, and part of the CH2 domain), the IgG Fc region was prepared using the CH2 domain and CH3 domain of wild-type IgG, and the IgM Fc region was using the cµ3 domain and cµ4 domain of wild-type IgM, and the J chain was prepared using the wild-type J chain.

Specifically, a recombinant plasmid was constructed to express the IgG Fc region (SEQ ID NO: 1, 2 or 3), the IgM Fc region (SEQ ID NO: 6), the linker (SEQ ID NO: 16) to be connected to the N-terminal part of the IgG Fc region, and the J chain (SEQ ID NO: 14) required for pentamer formation.

For gene cloning, after codon optimization of each of the material, codon-optimized nucleotides were synthesized in Cosmogenetech Inc. (Seoul, Korea). Insert DNA was prepared so that overlap PCR could be performed on the multiple cloning site (MCS) of pcDNA 3.1 (available from Thermo Scientific) vector using the optimized nucleotides and primers. After overlap PCR of the obtained insert DNAs and pcDNA 3.1 vectors, they were treated with DpnI restriction enzyme to remove the template, and each vector inserting each material was prepared.

After introducing the vector into Stella^{®} competent cell (available from Clontech), it was plated on an agar plate adding ampicillin antibiotics corresponding to the antibiotic resistance gene present in the vector, and then colonies were selected. The nucleotide sequence was confirmed through DNA sequencing using Applied biosystems 3730x1 DNA analyzer (available from Thermo Scientific).

### Example 2-2. Preparation of mutant fusion protein platform

In this Example 2-2, recombinant plasmids of linker, IgG Fc region, IgM Fc region and J chain were prepared, and a fusion protein in which the IgM Fc region and J chain were mutated was prepared. Based on the plasmid vector constructed in Example 2-1, some amino acids in the wild-type IgM domain were deleted or substituted, and some amino acids in the J chain were also deleted or substituted.

Specifically, random point mutations were made in the cµ3 and cµ4 domains (SEQ ID NO: 6) of the IgM Fc region, and in the wild-type J chain (SEQ ID NO: 14) to construct a recombinant plasmid.

For gene cloning, primers were synthesized in Cosmogenetech Inc. (Seoul, Korea). After performing the overlap PCR on the multiple cloning site (MCS) of pcDNA 3.1 (available from Thermo Scientific) vector using the primers, they were treated with DpnI restriction enzyme to remove the template, and each vector inserting each material was prepared.

After introducing the vector into Stella^{®} competent cell (available from Clontech), it was plated on an agar plate adding ampicillin antibiotics corresponding to the antibiotic resistance gene present in the vector, and then colonies were selected. The nucleotide sequence was confirmed through DNA sequencing using Applied biosystems 3730x1 DNA analyzer (available from Thermo Scientific).

### Example 2-3. Preparation of fusion protein comprising biologically active molecule

In this Example 2-3, a fusion protein comprising a biologically active molecule was prepared.

Specifically, after preparing the recombinant plasmid of the biologically active molecule, it was linked to the N terminus of the recombinant plasmid for fusion protein prepared in Example 2-1 or 2-2. The fusion protein was expressed and purified using the plasmid prepared in this way.

HLA-A or IgG antibody fragment (IgG Fab) was used as an example of a biologically active molecule.

### Example 2-3-1. Preparation of fusion protein comprising HLA-A

A fusion protein comprising HLA-A as a biologically active molecule, linker, IgG Fc region, IgM Fc region and J chain was prepared.

Specifically, in order to increase the stability of the HLA-A protein, cytomegalovirus (CMV)-derived peptide was additionally bound (loaded), and beta-2 microglobulin (β2m) was subsequently bound to the CMV-derived peptide. Accordingly, an CMVpHLA protein with improved stability was constructed, and its specific amino acid sequence is described in SEQ ID NO: 22.

The final recombinant plasmid vector of the fusion protein (hereinafter referred to as 'CMVpHLA fusion protein') comprising the biologically active molecule CMVpHLA constructed above and the linker, IgG Fc region, IgM Fc region and J chain was introduced into Expi293 (available from Thermo Scientific) animal cells to establish a transient expression system for CMVpHLA fusion protein.

CMVpHLA fusion protein plasmid DNA and J chain plasmid DNA were transfected at a ratio of 10:1, respectively. A total amount of 1 µg/mL of each recombinant plasmid vector was mixed with Opti-MEM^{®} medium, and ExpiFectamine^{®} reagent, and Opti-MEM^{®} medium were mixed and left at room temperature for 5 minutes. After that, DNA and ExpiFectamine^{®} reagent were reacted for 15 minutes and added to the flask. They were treated with enhancer 1 and 2 after 16 to 22 hours. After confirming 75% or more of cell viability on the day 4 or 5, a fusion protein was recovered.

Then, the expressed CMVpHLA fusion protein was purified using IgM affinity chromatography. Specifically, the culture solution obtained from the transient expression system was loaded onto the IgM affinity matrix POROS^{™} CaptureSelect^{™} IgM Affinity Matrix^{®} (available from Thermo Scientific) to purify the fusion protein. IgM affinity chromatography was performed under the following conditions:

### <Chromatography Conditions>

- Resin: POROS^{™} CaptureSelect^{™} IgM Affinity Matrix^{®}
- Flow rate: 480 cm/h
- Equilibration: Phosphate-buffered saline (PBS) buffer
- Loading: up to 6 g protein/L resin volume
- Regeneration and sterilization: 0.01 M NaOH solution
- Elution: 0.1 M glycine-HCl, pH 2.7 buffer
- Storage: 20% EtOH

Then, it was neutralized with 1.0 M Tris-HCl, pH 8.5 buffer in an amount of 1/10 of the volume of the eluate. The recovered CMVpHLA fusion protein was concentrated using ultrafiltration, dialyzed against PBS, and then stored at -20 °C.

The CMVpHLA fusion protein prepared by the above-described method is indicated as follows. The fusion protein was expressed as the structural formula starting from the N-terminus: [(biologically active molecule)-(linker)-(IgG Fc region)-(IgM Fc region)]-(J chain). For variants, amino acids substituted for existing amino acids in the corresponding region were indicated based on the numbering of SEQ ID NO: 6 of IgM and SEQ ID NO: 14 of the J chain. Hereafter, the CMVpHLA fusion protein is named as shown in Table 7 below. In the case of fusion proteins containing random point mutations, they were not named separately if there were problems with expression or if a pentamer was not formed.
- Wild-type IgM Fc region sequence (SEQ ID NO: 6, wherein the 124th and/or 125th amino acids underlined and in bold may contain mutations): 
QDTAIRVFAI PPSFASIFLT KSTKLTCLVT DLTTYDSVTI SWTRQNGEAV
KTHTNISESH PNATFSAVGE ASICEDDWNS GERFTCTVTH TDLPSPLKQT
ISRPKGVALH RPDVYLLPPA REQLNLRESA TITCLVTGFS PADVFVQWMQ
RGQPLSPEKY VTSAPMPEPQ APGRYFAHSI LTVSEEEWNT GETYTCVVAH
EALPNRVTER TVDKSTGKPT LYNVSLVMSD TAGTCY
- Wild-type J chain sequence (SEQ ID NO: 14, wherein the 105th, 106th and/or 107th amino acids underlined and in bold may contain mutations):

QEDERIVLVD NKCKCARITS RIIRSSEDPN EDIVERNIRI IVPLNNRENI SDPTSPLRTR

FVYHLSDLCK KCDPTEVELD NQIVTATQSN ICDEDSATET CYTYDRNKCY

TAVVPLVYGG ETKMVETALT PDACYPD

**[Table 7]**

| **CMVpHLA fusion protein** | **Name** |
|---|---|
| (CMVpHLA)₂-(IgG Fc region) | IMB-4000 |
| [(CMVpHLA)₂-(linker)₂-(IgM Fc region)]₅-(J chain) | IMB-4001 |
| [(CMVpHLA)₂-(linker)₂-(IgG Fc region)-(IgM Fc region)]s-(J chain) | IMB-4011 |
| [(CMVpHLA)₂-(linker)₂-(IgG Fc region)-(IgM Fc region)]s-(J chain mutant) | IMB-4012 |
| [(CMVpHLA)₂-(linker)₂-(IgG Fc region)-(IgM Fc region N124A)]s-(J chain) | IMB-4012-1 |
| [(CMVpHLA)₂-(linker)₂-(IgG Fc region)-(IgM Fc region N124F)]s-(J chain) | IMB-4012-2 |
| [(CMVpHLA)₂-(linker)₂-(IgG Fc region)-(IgM Fc region N124K)]s-(J chain) | IMB-4012-3 |
| [(CMVpHLA)₂-(linker)₂-(IgG Fc region)-(IgM Fc region L125 deletion)]₅-(J chain) | IMB-4012-4 |
| [(CMVpHLA)₂-(linker)₂-(IgG Fc region)-(IgM Fc region L125A)]s-(J chain) | IMB-4012-5 |
| [(CMVpHLA)₂-(linker)₂-(IgG Fc region)-(IgM Fc region L125D)]s-(J chain) | IMB-4012-6 |
| [(CMVpHLA)₂-(linker)₂-(IgG Fc region)-(IgM Fc region L125E)]₅-(J chain) | IMB-4012-7 |
| [(CMVpHLA)₂-(linker)₂-(IgG Fc region)-(IgM Fc region L125F)]s-(J chain) | IMB-4012-8 |
| [(CMVpHLA)₂-(linker)₂-(IgG Fc region)-(IgM Fc region L125G)]₅-(J chain) | IMB-4012-9 |
| [(CMVpHLA)₂-(linker)₂-(IgG Fc region)-(IgM Fc region L125H)]s-(J chain) | IMB-4012-10 |
| [(CMVpHLA)₂-(linker)₂-(IgG Fc region)-(IgM Fc region L125I)]s-(J chain) | IMB-4012-11 |
| [(CMVpHLA)₂-(linker)₂-(IgG Fc region)-(IgM Fc region L125K)]s-(J chain) | IMB-4012-12 |
| [(CMVpHLA)₂-(linker)₂-(IgG Fc region)-(IgM Fc region L125M)]₅-(J chain) | IMB-4012-13 |
| [(CMVpHLA)₂-(linker)₂-(IgG Fc region)-(IgM Fc region L125N)]₅-(J chain) | IMB-4012-14 |
| [(CMVpHLA)₂-(linker)₂-(IgG Fc region)-(IgM Fc region L125P)]₅-(J chain) | IMB-4012-15 |
| [(CMVpHLA)₂-(linker)₂-(IgG Fc region)-(IgM Fc region L125Q)]s-(J chain) | IMB-4012-16 |
| [(CMVpHLA)₂-(linker)₂-(IgG Fc region)-(IgM Fc region L125R)]₅-(J chain) | IMB-4012-17 |
| [(CMVpHLA)₂-(linker)₂-(IgG Fc region)-(IgM Fc region L125S)]₅-(J chain) | IMB-4012-18 |
| [(CMVpHLA)₂-(linker)₂-(IgG Fc region)-(IgM Fc region L125T)]s-(J chain) | IMB-4012-19 |
| [(CMVpHLA)₂-(linker)₂-(IgG Fc region)-(IgM Fc region L125V)]s-(J chain) | IMB-4012-20 |
| [(CMVpHLA)₂-(linker)₂-(IgG Fc region)-(IgM Fc region L125W)]s-(J chain) | IMB-4012-21 |
| [(CMVpHLA)₂-(linker)₂-(IgG Fc region)-(IgM Fc region L125Y)]₅-(J chain) | IMB-4012-22 |
| [(CMVpHLA)₂-(linker)₂-(IgG Fc region)-(IgM Fc region)]₅-(J chain D105A) | IMB-4012-23 |
| [(CMVpHLA)₂-(linker)₂-(IgG Fc region)-(IgM Fc region)]₅-(J chain D105K) | IMB-4012-24 |
| [(CMVpHLA)₂-(linker)₂-(IgG Fc region)-(IgM Fc region)]s-(J chain R106 deletion) | IMB-4012-25 |
| [(CMVpHLA)₂-(linker)₂-(IgG Fc region)-(IgM Fc region)]s-(T chain R106A) | IMB-4012-26 |
| [(CMVpHLA)₂-(linker)₂-(IgG Fc region)-(IgM Fc region)]₅-(J chain R106D) | IMB-4012-27 |
| [(CMVpHLA)₂-(linker)₂-(IgG Fc region)-(IgM Fc region)]s-(J chain R106E) | IMB-4012-28 |
| [(CMVpHLA)₂-(linker)₂-(IgG Fc region)-(IgM Fc region)]s-(J chain R106F) | IMB-4012-29 |
| [(CMVpHLA)₂-(linker)₂-(IgG Fc region)-(IgM Fc region)]s-(J chain R106G) | IMB-4012-30 |
| [(CMVpHLA)₂-(linker)₂-(IgG Fc region)-(IgM Fc region)]s-(J chain R106H) | IMB-4012-31 |
| [(CMVpHLA)₂-(linker)₂-(IgG Fc region)-(IgM Fc region)]s-(J chain R106I) | IMB-4012-32 |
| [(CMVpHLA)₂-(linker)₂-(IgG Fc region)-(IgM Fc region)]s-(J chain R106K) | IMB-4012-33 |
| [(CMVpHLA)₂-(linker)₂-(IgG Fc region)-(IgM Fc region)]s-(J chain R106L) | IMB-4012-34 |
| [(CMVpHLA)₂-(linker)₂-(IgG Fc region)-(IgM Fc region)]s-(J chain R106M) | IMB-4012-35 |
| [(CMVpHLA)₂-(linker)₂-(IgG Fc region)-(IgM Fc region)]s-(J chain R106N) | IMB-4012-36 |
| [(CMVpHLA)₂-(linker)₂-(IgG Fc region)-(IgM Fc region)]s-(J chain R106P) | IMB-4012-37 |
| [(CMVpHLA)₂-(linker)₂-(IgG Fc region)-(IgM Fc region)]s-(J chain R106) | IMB-4012-38 |
| [(CMVpHLA)₂-(linker)₂-(IgG Fc region)-(IgM Fc region)]s-(J chain R106S) | IMB-4012-39 |
| [(CMVpHLA)₂-(linker)₂-(IgG Fc region)-(IgM Fc region)]s-(J chain R106T) | IMB-4012-40 |
| [(CMVpHLA)₂-(linker)₂-(IgG Fc region)-(IgM Fc region)]s-(J chain R106V) | IMB-4012-41 |
| [(CMVpHLA)₂-(linker)₂-(IgG Fc region)-(IgM Fc region)]s-(J chain R106W) | IMB-4012-42 |
| [(CMVpHLA)₂-(linker)₂-(IgG Fc region)-(IgM Fc region)]s-(J chain R106Y) | IMB-4012-43 |
| [(CMVpHLA)₂-(linker)₂-(IgG Fc region)-(IgM Fc region)]s-(J chain N107A) | IMB-4012-44 |
| [(CMVpHLA)₂-(linker)₂-(IgG Fc region)-(IgM Fc region)]s-(J chain N107E) | IMB-4012-45 |
| [(CMVpHLA)₂-(linker)₂-(IgG Fc region)-(IgM Fc region)]s-(J chain N107F) | IMB-4012-46 |
| [(CMVpHLA)₂-(linker)₂-(IgG Fc region)-(IgM Fc region)]s-(J chain N107K) | IMB-4012-47 |
| [(CMVpHLA)₂-(linker)₂-(IgG Fc region)-(IgM Fc region L125G)]s-(J chain R106V) | IMB-4012-48 |
| [(CMVpHLA)₂-(linker)₂-(IgG Fc region)-(IgM Fc region L125G)]s-(J chain R106W) | IMB-4012-49 |
| [(CMVpHLA)₂-(linker)₂-(IgG Fc region)-(IgM Fc region L125S)]s-(J chain R106V) | IMB-4012-50 |
| [(CMVpHLA)₂-(linker)₂-(IgG Fc region)-(IgM Fc region L125S)]s-(J chain R106W) | IMB-4012-51 |

Meanwhile, as a control, a fusion protein not comprising IgM Fc region (hereinafter referred to as 'CMVpHLA-IgG Fc') was prepared. As the IgG Fc region comprised in the fusion protein, the CH2 domain and CH3 domain of IgG were used. Purification and concentration of the control group were performed as follows. The culture solution obtained from the transient expression system was loaded onto the IgG affinity matrix MabSelect SuRe^{®} (available from Cytiva) and purified.

IgG affinity chromatography was performed under the following conditions:

### <Chromatography Conditions>

- Resin: MabSelect SuRe^{®}
- Flow rate: 300 cm/h
- Equilibration: Phosphate-buffered saline (PBS) buffer
- Loading: up to 35 g protein/L resin volume
- Regeneration and sterilization: 0.01 M NaOH solution
- Elution: 0.1 M glycine-HCl, pH 2.7 buffer
- Storage: 20% EtOH

After the IgG affinity chromatography, it was neutralized with 1.0 M Tris-HCl, pH 8.5 buffer in an amount of 1/10 of the volume of the eluate. The recovered fusion protein was concentrated using ultrafiltration, dialyzed against PBS, and then stored at -20 °C.

Through this, it was confirmed that the binding affinity with pIgR decreased, and accordingly the half-life in the blood increased. In addition, the fusion protein containing the mutation showed a reduced binding affinity to pIgR compared to the fusion protein (IMB-4001 and IMB-4011) not containing the mutation.

### Example 2-3-2. Preparation of fusion protein comprising IgG antibody fragment

A fusion protein (hereinafter referred to as 'A2014 fusion protein') comprising an IgG antibody fragment (IgG Fab; hereinafter referred to as 'A2014') as a biologically active molecule, linker, IgG Fc region, IgM Fc region and J chain was prepared.

The A2014 was prepared using the light chain (VL domain and CL domain; SEQ ID NO: 27) and the part of heavy chain (VH domain and CH1 domain; SEQ ID NO: 31) among the IgG Fab moieties by the cloning method according to Example 2-1.

A fusion protein not comprising IgM Fc region (hereinafter referred to as 'A2014-IgG') was prepared as a control. As the IgG Fc region comprised in A2014-IgG, the CH2 and CH3 domains of IgG were used, specifically the light chain (VL domain and CL domain; SEQ ID NO: 27) and the heavy chain (VH domain, CH1 domain, CH2 domain and CH3 domain; SEQ ID NO: 29). It was prepared using the cloning method according to Example 2-1.

The constructed A2014 recombinant plasmid sequence was linked to the fusion protein platform plasmid sequence by the method according to Example 2-3-1, and the final recombinant plasmid vector thus constructed was introduced into cells and each protein was expressed.

Specifically, plasmid DNAs of a portion of the A2014 heavy chain, A2014 light chain and J chain were transfected at a ratio of 4.5:4.5:1, respectively. A total amount of 1 µg/mL of each recombinant plasmid vector was mixed with Opti-MEM^{®} medium, and ExpiFectamine^{®} reagent, and Opti-MEM^{®} medium were mixed and left at room temperature for 5 minutes. After that, DNA and ExpiFectamine^{®} reagent were reacted for 15 minutes and added to the flask. They were treated with enhancer 1 and 2 after 16 to 22 hours. After confirming 75% or more of cell viability on the day 4 or 5, a fusion protein was recovered.

Thereafter, the culture solution was purified and concentrated, dialyzed against PBS, and stored at -20 °C in the same manner as in Example 2-3-1.

The A2014 fusion protein prepared by the above-described method is expressed as a structural formula as shown in Table 7, and is named as shown in Table 8.

**[Table 8]**

| **A2014 fusion protein** | **Name** |
|---|---|
| (A2014)₂-(IgG Fc region) | A2014-1 |
| [(A2014)₂-(linker)-(IgM Fc region)]₅-(J chain) | A2014-2 |
| [(A2014)₂-(linker)-(IgG Fc region)-(IgM Fc region)]s-(J chain) | A2014-3 |
| [(A2014)₂-(linker)-(IgG Fc region)-(IgM Fc region)]s-(J chain mutant) | A2014-4 |

### Example 2-4. Structure of the prepared fusion protein

It was confirmed whether the materials prepared in Examples 2-3-1 and 2-3-2 have the desired structure.

A schematic diagram of the pentameric fusion protein comprising biologically active molecule, linker, IgG Fc region, IgM Fc variant and J chain variant is shown in FIG. 11B.

Specifically, the structure of the fusion protein was confirmed using Reducing SDS-PAGE. After mixing 5x reducing-PAGE sample buffer with 1 µg of each fusion protein, it was heated at 100 °C for 5 minutes, and then left at room temperature for 20 minutes. After inserting polyamide gel (12-20%, Invitrogen) into the gel kit, loading the sample, and electrophoresing it at 200V for 30 minutes, the gel was separated from the kit and stained with Coomassie Brilliant Blue staining solution for 1 hour. Then, the gel was transferred to a buffer (destaining buffer; 70% tertiary distilled water, 20% methanol, 10% acetic acid), and the result was confirmed after overnight.

As a result, as shown in FIGs. 12A and 12B, each of the fusion protein comprising biologically active molecule, linker, IgG Fc region, IgM Fc region and J chain was confirmed to contain all sizes corresponding to components that constitute it.

The results show that the fusion proteins prepared in Example 2-3 have the desired structure and are normally expressed.

### Example 2-5. Biological activity of fusion protein

In this Example 2-5, the biological activity of the fusion protein prepared in Example 2-3 was analyzed. The purpose of this test is to determine whether the effect of increasing the target binding activity shown in the fusion protein without the mutation is maintained in the fusion protein containing the mutation. The target was set differently depending on the biologically active molecule (CMVpHLA or IgG antibody fragment) comprised in each fusion protein.

### Example 2-5-1. Proliferation of memory CD8₊ T cells by CMVpHLA fusion protein

In order to confirm the biological activity of the fusion protein prepared in Example 2-3-1, its ability to induce memory cell proliferation for CD8⁺ T cells was analyzed.

The ability to induce memory cell proliferation for CD8⁺ T cells was confirmed through FACS. With approval from the Institutional Review Board, PBMCs were isolated from human donor blood obtained from the Gyeonggi Red Cross using Ficoll gradient centrifugation. The PBMC 1×10⁶ cells/mL were suspended in RPMI-1640 (10% FBS) and seeded in 6-well at a density of 1×10⁶ cells/well. Afterwards, CMVpHLA fusion protein or control protein was treated at a concentration of 100 nM per well, and IL-2 (available from Peprotech) was added at 20 U/mL and cultured. After 3 to 4 days, 20 U/mL of IL-2 was added, and on the 7th to 8th day of culture, half of the starting volume of culture medium was added along with each protein and IL-2. On days 13 to 14, each cell was harvested and washed with FACS buffer (5% FBS in PBS).

Thereafter, the cell suspension was treated with an isotype antibody (available from Biolegend) to perform Fc blocking, and then washed with FACS buffer. Afterwards, it was treated with PerCP cy5.5 anti-human CD8 antibody (available from BD bioscience), APC anti-human CD45RA antibody (available from BD bioscience) and FITC anti-human CCR7 antibody (available from Biolegen), and then washed. After completing the process, the degree of memory CD8⁺ T cell proliferation was confirmed using a flow cytometer (available from BD bioscience, FACSLyric^{™}).

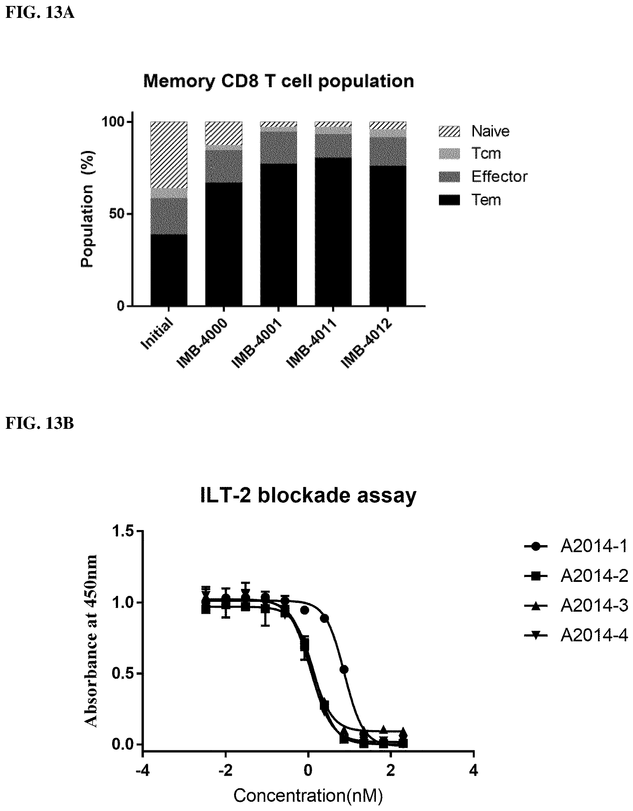

As a result, as shown in FIG. 13A, the percentage of naive CD8⁺ T cells on the initial starting day was analyzed to be 36%. For the CMVpHLA fusion proteins IMB-4001, IMB-4011 and IMB-4012, the proportion of naive CD8⁺ T cells was measured to be about 3% to 4%, and the remaining 30% or more converted into memory CD8⁺ T cells. However, the control group IMB-4000 (CMVpHLA-IgG) had a low binding activity, showing 13% of naive CD8⁺ T cells remained, which was analyzed to indicate a low conversion rate to memory CD8⁺ T cells.

The results suggest that the effect of increasing target binding activity seen in IMB-4001 and IMB-4011, which are fusion proteins without mutations, is also maintained in IMB-4012, a fusion protein containing mutations.

### Example 2-5-2. Activity blocking the binding of ILT-2 protein

To confirm the biological activity of the A2014 fusion protein prepared in Example 2-3-2 and the control group, the activity blocking the binding of an HLA-G protein and a ILT-2 (LILRB1) protein was analyzed. The ILT-2 protein is a receptor protein expressed in immune cells, and when binding to the HLA-G protein, the activities of cytotoxic T cells, NK cells and B cells are suppressed, so that a normal immune response is not occurred.

To confirm the biological activity of the fusion protein, the activity blocking the binding between a recombinant HLA-G protein and a recombinant ILT-2 protein was analyzed by ELISA assay. ILT-2 antigen (R&D systems) was put in a 96-well plate at 150 ng/well, and then coated at 4°C for 16 hours. A plate was washed with 0.05% PBS-Tween and blocked with 3% skim milk. After blocking, a dilution containing each A2014 fusion protein, A2014 variant fusion protein or control protein was added to the well, and HLA-G-6x his tag (IMBiologics) was treated at a predetermined concentration in each well and incubated at 37°C for 2 hours. Then, the plate was washed, and HRP-conjugated anti-6x his antibody (available from abcam) was diluted 1:10,000 and incubated at 37 °C for 1 hour. Thereafter, the plate was washed with 0.05% PBS-Tween, developed with TMB solution (available from Sigma), and then the reaction was terminated with 1N sulfuric acid (available from Daejung Chemicals & Metals) and analyzed by VARIOSKAN LUX (available from Thermo Scientific).

As a result, as shown in FIG. 13B, the A2014-1 (A2014-IgG) control group had a very low activity in blocking the binding of the recombinant HLA-G protein and the ILT-2 protein. On the contrary, the A2014 fusion protein (A2014-2, A2014-3, A2014-4) had a very high activity in blocking binding of the proteins, and the IC₅₀ value was improved about 5 times or more compared to the control protein.

The results suggest that the effect of increasing target binding activity seen in A2014-2 and A2014-3, which are fusion proteins without mutations, is also maintained in IMB-4012, a fusion protein comprising mutations.

### Example 2-6. Receptor affinity of fusion protein

### Example 2-6-1. Affinity for FcRn interaction

In order to confirm whether the fusion protein prepared in Example 2-3 increases the half-life in vivo through binding to FcRn, an ELISA analysis was performed to check the interaction with FcRn. Binding with the FcRn must occur in the vicinity of mild acidity (<pH 6.5), and binding must not occur in neutral (pH 7.4) to increase the half-life for recycling to occur.

Specifically, to confirm binding ability with FcRn, 1 ug/well of the conjugate of FcRn and β2m antigen (ACRO biosystems) was added to a 96-well plate and coated at 4°C for 16 hours. All solutions confirming binding under mildly acidic conditions were prepared at pH 6.0, and all solutions confirming binding under neutral conditions were prepared at pH 7.4. Subsequently, the plate was washed with 0.05% PBS-Tween and blocked with 3% skim milk. After blocking, dilutions comprising each fusion protein, variant fusion protein, or control protein were added to the wells and incubated at 37°C for 2 hours. Next, the plate was washed, and HRP-linked anti-IgM (F5µ) antibody (MERCK) or anti-IgG Fc antibody (Invitrogen) was diluted 1:5,000 and incubated at 37°C for 1 hour. Afterwards, the plate was washed with 0.05% PBS-Tween, developed with TMB solution (Sigma), and the reaction was terminated with 1N sulfuric acid (Daejung Chemical) and analyzed with VARIOSKAN LUX (Thermo Scientific).

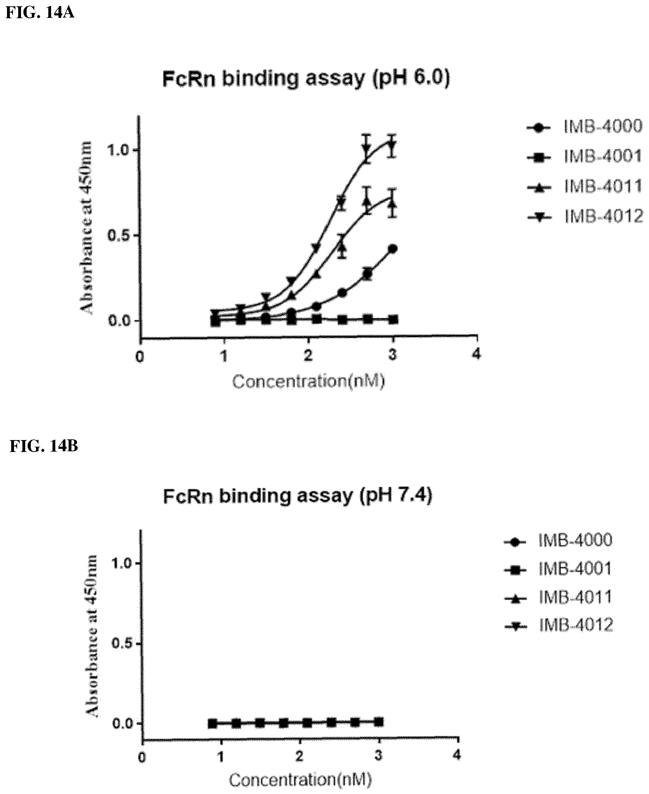

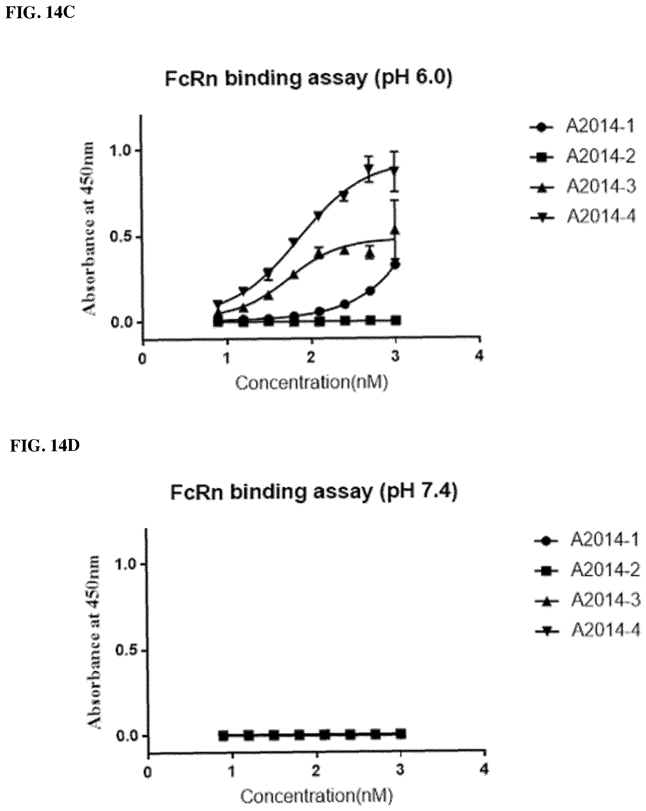

As a result, as shown in FIGs. 14a to 14d, the FcRn binding affinity of the fusion protein without IgG Fc (IMB-4001 or A2014-1) was significantly lower than that of the fusion protein containing IgG Fc (IMB-4011 or A2014-3; IMB-4012 or A2014-4).

On the other hand, the fusion protein comprrising IgG Fc (IMB-4011 or A2014-3; IMB-4012 or A2014-4) has a very high binding affinity to FcRn compared to the control group, especially the fusion protein containing a mutation (IMB-4012 or A2014-4) was found to have better binding efficacy than fusion proteins (IMB-4011 or A2014-3) that do not contain mutations.

The results show that the fusion protein has an advantage in binding efficacy with FcRn due to the introduction of the IgG Fc region and mutations induced in the IgM Fc region and J chain, thereby improving the strength of interaction and increasing FcRn recycling efficiency.

### Example 2-6-2. Affinity for pIgR (polymeric Ig Receptor) interaction

In order to confirm whether the fusion protein prepared in Example 2-3 had a reduced binding affinity to pIgR and increased half-life in the blood, an ELISA analysis was performed to check the interaction with pIgR.

Specifically, to confirm binding ability with pIgR, 100 ng/well of pIgR (R&D systems) was added to a 96-well plate and coated at 4°C for 16 hours. Subsequently, the plate was washed with 0.05% PBS-Tween and blocked with 3% skim milk. After blocking, dilutions comprising each fusion protein and variant fusion protein were added to the wells and incubated at 37°C for 2 hours. Next, the plate was washed, and HRP-linked anti-IgM (F5µ) antibody (MERCK) was diluted 1:5,000 and incubated at 37°C for 1 hour. Afterwards, the plate was washed with 0.05% PBS-Tween, developed with TMB solution (Sigma), and the reaction was terminated with 1N sulfuric acid (Daejung Chemical) and analyzed with VARIOSKAN LUX (Thermo Scientific).

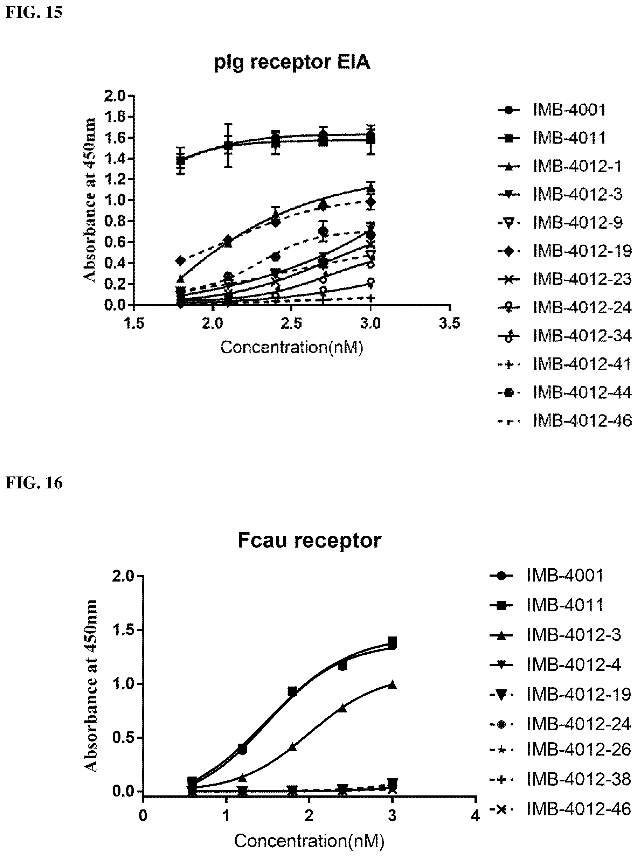

As a result, as shown in FIG 15, when mutations occur in N124 of the IgM Fc region, L125 of the IgM Fc region, D105 of the J chain, R106 of the J chain, and/or N107 of the J chain, the binding affinity with pIgR decreased compared to the wild-type fusion protein (IMB-4001, IMB-4011) which does not cause mutation. In detail, Table 9 lists the relative binding force values when the binding force between IMB-4011 and pIgR is assumed to be 100%. When the amino acids in the corresponding five regions were replaced, differences in binding ability to pIgR occurred depending on the converted amino acid. In addition, the difference in binding strength does not have a certain tendency depending on the group classified according to the charge, polarity, and functional group of the amino acid, so changes in binding strength due to specific amino acids were confirmed only experimentally.

**[Table 9]**

| **fusion protein** | **Relative binding ability to plgR (%)** |
|---|---|
| IMB-4001 | 102 |
| IMB-4011 | 100 |
| IMB-4012-1 | 63 |
| IMB-4012-2 | 57 |
| IMB-4012-3 | 30 |
| IMB-4012-4 | 6 |
| IMB-4012-5 | 62 |
| IMB-4012-6 | 50 |
| IMB-4012-7 | 79 |
| IMB-4012-8 | 84 |
| IMB-4012-9 | 24 |
| IMB-4012-10 | 61 |
| IMB-4012-11 | 86 |
| IMB-4012-12 | 86 |
| IMB-4012-13 | 91 |
| IMB-4012-14 | 72 |
| IMB-4012-15 | 72 |
| IMB-4012-16 | 92 |
| IMB-4012-17 | 60 |
| IMB-4012-18 | 8 |
| IMB-4012-19 | 58 |
| IMB-4012-20 | 82 |
| IMB-4012-21 | 90 |
| IMB-4012-22 | 88 |
| IMB-4012-23 | 25 |
| IMB-4012-24 | 8 |
| IMB-4012-25 | 4 |
| IMB-4012-26 | 5 |
| IMB-4012-27 | 5 |
| IMB-4012-28 | 4 |
| IMB-4012-29 | 4 |
| IMB-4012-30 | 3 |
| IMB-4012-31 | 4 |
| IMB-4012-32 | 3 |
| IMB-4012-33 | 3 |
| IMB-4012-34 | 11 |
| IMB-4012-35 | 4 |
| IMB-4012-36 | 3 |
| IMB-4012-37 | 4 |
| IMB-4012-38 | 3 |
| IMB-4012-39 | 3 |
| IMB-4012-40 | 4 |
| IMB-4012-41 | 4 |
| IMB-4012-42 | 4 |
| IMB-4012-43 | 4 |
| IMB-4012-44 | 45 |
| IMB-4012-45 | 20 |
| IMB-4012-46 | 3 |
| IMB-4012-47 | 8 |

Through this, it was confirmed that the fusion protein with mutations in the IgM Fc region and/or J chain had a reduced binding affinity to pIgR through mutation, and increased half-life in the blood. In addition, the fusion protein comprising the mutation had reduced binding affinity to pIgR compared to the fusion protein (IMB-4001 and IMB-4011) which did not comprise the mutation.

### Example 2-6-3. Affinity for Fcα/µ interaction

In order to confirm whether non-specific binding to Fcα/µ receptors and half-life in the blood were increased in substances with effectively reduced binding affinity to pIgR among the fusion proteins prepared in Example 2-3, ELISA analysis was performed.

Specifically, to confirm binding ability with Fcα/µ, 100 ng/well of Fcα/µ (R&D systems) was added to a 96-well plate and coated at 4°C for 16 hours. Subsequently, the plate was washed with 0.05% PBS-Tween and blocked with 3% skim milk. After blocking, dilutions comprising each fusion protein and variant fusion protein were added to the wells and incubated at 37°C for 2 hours. Next, the plate was washed, and HRP-linked anti-IgM (F5µ) antibody (MERCK) was diluted 1:5,000 and incubated at 37°C for 1 hour. Afterwards, the plate was washed with 0.05% PBS-Tween, developed with TMB solution (Sigma), and the reaction was terminated with 1N sulfuric acid (Daejung Chemical) and analyzed with VARIOSKAN LUX (Thermo Scientific).

As a result, as shown in FIG 16, when mutations occur in N124 of the IgM Fc region, L125 of the IgM Fc region, D105 of the J chain, R106 of the J chain, and/or N107 of the J chain, the binding affinity with Fcα/µ decreased compared to the wild-type fusion protein (IMB-4001, IMB-4011) which does not cause mutation. In detail, Table 10 lists the relative binding force values when the binding force between IMB-4011 and Fcα/µ is assumed to be 100%. When the amino acids in the corresponding five regions were replaced, differences in binding ability to Fcα/µ occurred depending on the converted amino acid. In addition, the difference in binding strength does not have a certain tendency depending on the group classified according to the charge, polarity, and functional group of the amino acid, so changes in binding strength due to specific amino acids were confirmed only experimentally.

**[Table 10]**

| **fusion protein** | **Relative binding ability to Fcα/µ receptor (%)** |
|---|---|
| IMB-4001 | 99 |
| IMB-4011 | 100 |
| IMB-4012-3 | 66 |
| IMB-4012-4 | 1 |
| IMB-4012-6 | 1 |
| IMB-4012-9 | 1 |
| IMB-4012-18 | 1 |
| IMB-4012-19 | 1 |
| IMB-4012-24 | 1 |
| IMB-4012-25 | 1 |
| IMB-4012-26 | 2 |
| IMB-4012-29 | 7 |
| IMB-4012-30 | 2 |
| IMB-4012-31 | 1 |
| IMB-4012-32 | 2 |
| IMB-4012-33 | 1 |
| IMB-4012-35 | 2 |
| IMB-4012-36 | 1 |
| IMB-4012-37 | 1 |
| IMB-4012-38 | 1 |
| IMB-4012-40 | 1 |
| IMB-4012-41 | 2 |
| IMB-4012-42 | 4 |
| IMB-4012-43 | 37 |
| IMB-4012-46 | 9 |

Through this, it was confirmed that the fusion protein with mutations in the IgM Fc region and/or J chain had a reduced binding affinity to Fcα/µ through mutation, and increased half-life in the blood. In addition, the fusion protein comprising the mutation had reduced binding affinity to Fcα/µ compared to the fusion protein (IMB-4001 and IMB-4011) which did not comprise the mutation.

### Example 2-6-4. Affinity for Fcµ interaction

In order to confirm whether non-specific binding to Fcµ receptors and half-life in the blood were increased in substances with effectively reduced binding affinity to pIgR among the fusion proteins prepared in Example 2-3, ELISA analysis was performed.

Specifically, to confirm binding ability with Fcµ, 100 ng/well of Fcµ (R&D systems) was added to a 96-well plate and coated at 4°C for 16 hours. Subsequently, the plate was washed with 0.05% PBS-Tween and blocked with 3% skim milk. After blocking, dilutions comprising each fusion protein and variant fusion protein were added to the wells and incubated at 37°C for 2 hours. Next, the plate was washed, and HRP-linked anti-IgM (F5µ) antibody (MERCK) was diluted 1:5,000 and incubated at 37°C for 1 hour. Afterwards, the plate was washed with 0.05% PBS-Tween, developed with TMB solution (Sigma), and the reaction was terminated with 1N sulfuric acid (Daejung Chemical) and analyzed with VARIOSKAN LUX (Thermo Scientific).

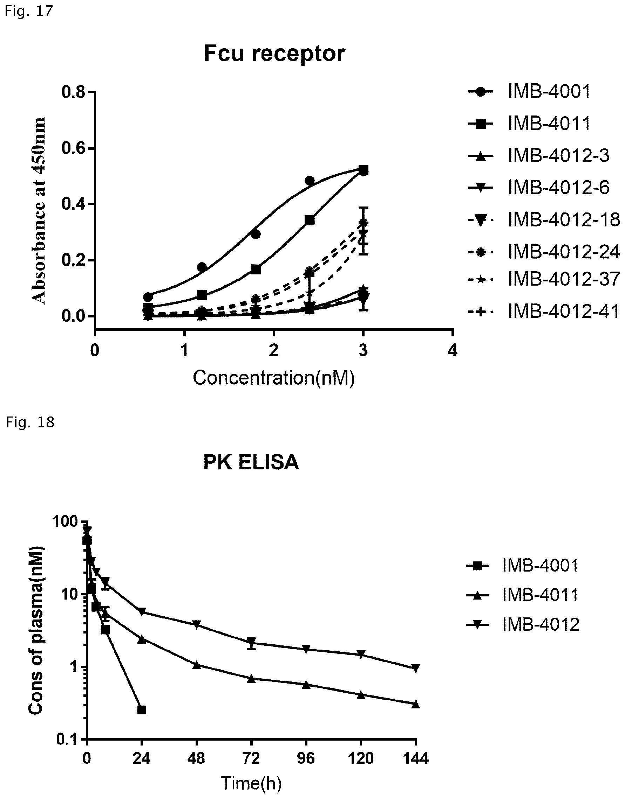

As a result, as shown in FIG 17, when mutations occur in N124 of the IgM Fc region, L125 of the IgM Fc region, D105 of the J chain, R106 of the J chain, and/or N107 of the J chain, the binding affinity with Fcµ decreased compared to the wild-type fusion protein (IMB-4001, IMB-4011) which does not cause mutation. In detail, Table 11 lists the relative binding force values when the binding force between IMB-4011 and Fcµ is assumed to be 100%. When the amino acids in the corresponding five regions were replaced, differences in binding ability to Fcµ occurred depending on the converted amino acid. In addition, the difference in binding strength does not have a certain tendency depending on the group classified according to the charge, polarity, and functional group of the amino acid, so changes in binding strength due to specific amino acids were confirmed only experimentally.

**[Table 11]**

| **fusion protein** | **Relative binding ability to Fcµ receptor (%)** |
|---|---|
| IMB-4001 | 142 |
| IMB-4011 | 100 |
| IMB-4012-3 | 7 |
| IMB-4012-4 | 12 |
| IMB-4012-6 | 11 |
| IMB-4012-9 | 7 |
| IMB-4012-18 | 7 |
| IMB-4012-19 | 11 |
| IMB-4012-24 | 34 |
| IMB-4012-25 | 59 |
| IMB-4012-26 | 68 |
| IMB-4012-29 | 50 |
| IMB-4012-30 | 44 |
| IMB-4012-31 | 54 |
| IMB-4012-32 | 70 |
| IMB-4012-33 | 57 |
| IMB-4012-35 | 53 |
| IMB-4012-36 | 47 |
| IMB-4012-37 | 39 |
| IMB-4012-38 | 86 |
| IMB-4012-40 | 92 |
| IMB-4012-41 | 69 |
| IMB-4012-42 | 72 |
| IMB-4012-43 | 111 |
| IMB-4012-46 | 34 |

Through this, it was confirmed that the fusion protein with mutations in the IgM Fc region and/or J chain had a reduced binding affinity to Fcµ through mutation, and increased half-life in the blood except for one mutation (IMB-4012-43). In addition, the fusion protein comprising the mutation had reduced binding affinity to Fcα/µ compared to the fusion protein (IMB-4001 and IMB-4011) which did not comprise the mutation. The results of show that when appropriately combining mutations in the IgM Fc region and mutations in the J chain, non-specific binding to the target of the fusion protein can be reduced and further, the half-life in the blood can be increased.

### Example 2-7. Affinity for IgM receptor interaction

In this Example 2-7, it was confirmed whether the binding affinity of the fusion proteins having mutations in both the IgM Fc region and J chain is reduced for all three IgM receptors.

Specifically, ELISA analysis for three types of IgM receptors was performed in the same manner as in Examples 4, 5, or 6 above. In detail, Table 9 lists the relative binding force values when the binding force between IMB-4011 and three IgM receptors is assumed to be 100%. In addition, the difference in binding strength does not have a certain tendency depending on the group classified according to the charge, polarity, and functional group of the amino acid, so changes in binding strength due to specific amino acids were confirmed only experimentally.

**[Table 12]**

| **Specifically, ELISA analysis for three types of IgM receptors was performed in the same manner as in Examples 4, 5, or 6 above.** | **Relative binding ability to pIgR (%)** | **Relative binding ability to Fcα/µ receptor (%)** | **Relative binding ability to Fcµ receptor (%)** |
|---|---|---|---|
| IMB-4011 | 100 | 100 | 100 |
| IMG-4012-48 | 2 | 0.4 | 3 |
| IMG-4012-49 | 2 | 0.5 | 3 |
| IMG-4012-50 | 2 | 1 | 3 |
| IMG-4012-51 | 3 | 1 | 3 |

As a result, as shown in Table 12 above, the binding affinity of the fusion protein comprising mutations in both the IgM Fc region and the J chain was reduced for all three types of IgM receptors. The results show that mutations in the IgM Fc region and mutations in the J chain can complement each other to reduce binding to the IgM receptor, which can reduce non-specific binding in vivo and maximize half-life in the blood.

### Example 2-8. Half-life of fusion protein in blood

In this Example 2-8, it was confirmed whether the half-life in the blood of the fusion proteins having mutations in both the IgM Fc region and J chain is reduced.

Specifically, after administering the fusion protein to rats, the amount of protein in plasma was measured. Half-life in the blood refers to the quantification of the time period during which 50% of the initial concentration remains while the protein circulates in the body. Pharmacokinetic parameters were calculated using PKsolver. The experimental animals used were 6-week-old rats. After an acclimatization period of 1 week, based on the body weight measured on the day of test substance administration, the dose for each individual was slowly administered intravenously at 100 nM/kg, taking into account the different molecular weights of each fusion protein. After administration, blood was collected from animals by orbital bleed at 0.08, 2, 4, 8, 24, 48, 72, 96, 120, and 144 hours. The collected blood was separated into plasma using centrifugation and analyzed based on the enclosed test method using an IgG ELISA kit (Abcam) and an IgM ELISA kit (Abcam).

As a result, as shown in FIG. 18, in the case of a fusion protein (IMB-4001) that does not contain an IgG Fc region, the half-life in the blood was measured to be 4 hours. The fusion protein (IMB-4011) comprising the IgG Fc region has an increased half-life in the blood to 29 hours because FcRn recycling is possible despite binding to the IgM receptor.

In particular, the fusion protein (IMB-4012), which comprises an IgG Fc region and a mutation that does not bind to the IgM receptor, had the highest half-life in the blood, measured at 59 hours. This suggests that the half-life in the blood is maximized because of the FcRn recycling and inhibitory effect of IgM receptor binding.

That is, the results of the above-described examples show that the fusion protein platform according to the present invention, including the IgG Fc region, the IgM Fc region, the J chain, and the linker, can increase the avidity of biologically active molecules comprised in the fusion protein. Furthermore, it suggests that the half-life in the blood can be increased by inhibiting the FcRn recycling of the fusion protein and inhibiting binding to the IgM-specific receptor. In addition, if a mutation occurs in the N124 of the IgM Fc region, L125 of the IgM Fc region, D105 of the J chain, R106 of the J chain, and/or N107 of the J chain comprised in the fusion protein, the aforementioned effects can be further increased.

From the above description, those skilled in the art to which the present invention pertains will understand that the present invention can be implemented in other specific forms without changing its technical idea or essential features. In this regard, it should be understood that the embodiments described above are illustrative in all respects and are not limited thereto. The scope of the present invention should be construed as including the meaning and scope of the patent claims described below rather than the detailed description above, and all changes or modified forms derived from the equivalent concept thereof are included in the scope of the present invention.

## Claims

1. A fusion protein represented by a following formula:
[(X)ₐ-(Y)_{b}-(IgG Fc region)_{c}-(IgM Fc region)]ₘ-(Z)ₙ
wherein
X is biologically active molecule,
Y is a linker,
IgG Fc region is a monomer,
IgM Fc region is a monomer,
Z is a J chain,
a is 1 or 2, b is 1 or 2, c is 0 or 1, m is 5 or 6, and n is 0 or 1.

2. The fusion protein of claim 1, wherein the IgG Fc region comprises at least one selected from a group consisting of CH2 domain and CH3 domain.

3. The fusion protein of claim 1, wherein the IgG Fc region is derived from IgG1, IgG2, IgG3 or IgG4.

4. The fusion protein of claim 1, wherein the IgG Fc region is derived from wild-type IgG or variant IgG.

5. The fusion protein of claim 4, wherein the wild-type IgG comprises the amino acid sequence of SEQ ID NO: 1 or 3.

6. The fusion protein of claim 4, wherein the variant IgG comprises the amino acid sequence of SEQ ID NO: 2.

7. The fusion protein of claim 1, wherein the IgM Fc region comprises at least one selected from a group consisting of Cµ2 domain, Cµ3 domain and Cµ4 domain.

8. The fusion protein of claim 1, wherein the IgM Fc region is derived from wild-type IgM or variant IgM.

9. The fusion protein of claim 8, wherein the variant IgM comprises the amino acid sequence of SEQ ID NO: 6.

10. The fusion protein of claim 8, wherein the variant IgM is deleted or substituted one or more of the amino acids at the 124th and 125th positions in the amino acid sequence of SEQ ID NO: 6.

11. The fusion protein of claim 8, wherein the variant IgM has any one mutation selected from the group consisting of N124A, N124F and N124K at the 124th position in the amino acid sequence of SEQ ID NO: 6.

12. The fusion protein of claim 8, wherein the variant IgM has any one mutation selected from the group consisting of L125A, L125D, L125E, L125F, L125G, L125H, L125I, L125K, L125M, L125N, L125P, L125Q, L125R, L125S, L125T, L125V, L125W and L125Y at the 125th position in the amino acid sequence of SEQ ID NO: 6.

13. The fusion protein of claim 1, wherein the J chain is derived from wild-type J chain or variant J chain.

14. The fusion protein of claim 13, wherein the wild-type J chain comprises the amino acid sequence of SEQ ID NO: 14.

15. The fusion protein of claim 13, wherein the variant J chain is deleted or substituted one or more of the amino acids at the 105th, 106th and 107th positions in the amino acid sequence of SEQ ID NO: 14.

16. The fusion protein of claim 13, wherein the variant J chain has any one mutation selected from the group consisting of D105A and D105K at the 105th position in the amino acid sequence of SEQ ID NO: 14.

17. The fusion protein of claim 13, wherein the variant J chain has any one mutation selected from the group consisting of R106A, R106D, R106E, R106F, R106G, R106H, R106I, R106K, R106L, R106M, R106N, R106P, R106Q, R106S, R106T, R106V, R106W and R106Y at the 106th position in the amino acid sequence of SEQ ID NO: 14.

18. The fusion protein of claim 13, wherein the variant J chain has any one mutation selected from the group consisting of N107A, N107E, N107F and N107K at the 107th position in the amino acid sequence of SEQ ID NO: 14.

19. The fusion protein of claim 1, wherein the J chain is one to which a protein molecule is additionally bound.

20. The fusion protein of claim 19, wherein the protein molecule is one or more selected from the group consisting of antibodies, antigen-binding fragments of antibody, antibody-drug conjugates, antibody-like molecules, antigen-binding fragments of antibody-like molecule, soluble proteins, membrane-bound proteins, ligands, receptors, virus-like particles, protein toxins, enzymes, co-stimulatory receptors, cytokines, and cell engagers.

21. The fusion protein of claim 1, wherein the biologically active molecule is one or more selected from the group consisting of antibodies, antigen-binding fragments of antibody, antibody-drug conjugates, antibody-like molecules, antigen-binding fragments of antibody-like molecule, soluble proteins, membrane-bound proteins, ligands, receptors, virus-like particles, protein toxins, chemokines, cytokines, and enzymes.

22. The fusion protein of claim 1, wherein the linker is at least one selected from a group consisting of IgD hinge region, IgA hinge region, IgG hinge region and GS linker.

23. The fusion protein of claim 22, wherein the hinge region comprises a part or all of hinge region, a part or all of CH1 domain, a part or all of CH2 domain, or any combination thereof.

24. The fusion protein of claim 22, wherein the GS linker is at least one selected from a group consisting of (GS)_{d}, (SG)_{d}, (GGGS)_{d}, (GGGGS)_{d}, GCGS(GGGS)_{d} and GCGGS(GGGGS)_{d}, and wherein c is an integer between 2 and 6.

25. A nucleic acid molecule encoding the fusion protein of claim 1.

26. A vector for expression of fusion protein, comprising the nucleic acid molecule of claim 25.

27. A host cell transformed with the vector of claim 26.

28. A method of preparing the fusion protein of claim 1, comprising:
transforming a host cell with the vector of claim 26.

29. A use of the fusion protein of claim 1 for preparing pharmaceuticals for diagnosis or treatment.

30. A pharmaceutical composition comprising the fusion protein of claim 1.
